# EUROPEAN PATENT APPLICATION

(11) **EP 4 273 142 A1**
(43) Date of publication of application: **08.11.2023**
(21) Application number: 22305675.5
(22) Date of filing: 06.05.2022
(51) Int. Cl.: C07D 403/06, A61P 35/00, A61K 49/00, G01N 33/48, A61K 31/4427

(54) **PICOLINATE CYCLAM PALLADIUM CHELATES AND USES THEREOF**

(71) Applicant: Université de Bretagne Occidentale, 29238 Brest (FR); Centre national de la recherche scientifique, 75016 Paris (FR); The South African Nuclear Energy Corporation Soc Limited, 0240 Pretoria (ZA)
(72) Inventor: TRIPIER, Raphaël, 29860 Kersaint-Plabennec (FR); LEBRIS, Nathalie, 29200 Brest (FR); PINEAU, Julie, 29200 Brest (FR); DRIVER, Cathryn, 0181 Pretoria (ZA); ZEEVAART, Jan Rijn, 0182 Florauna (ZA)
(74) Representative: Icosa

(57) **Abstract**

The present invention relates to a chelate resulting from the complexation of a palladium(II) cation with a ligand of formula (I) 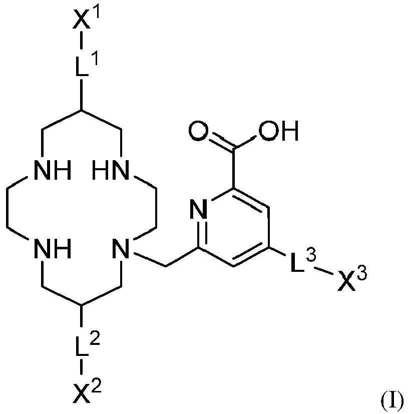 or a pharmaceutically acceptable salt and/or solvates thereof.

The invention further relates to the use of the chelate of the invention as diagnostic, therapeutic or theranostic agent, in particular for the diagnosis and/or the treatment of proliferative diseases.

The invention further relates to a process for manufacturing a chelate of the invention.

## Description

### FIELD OF INVENTION

The present invention relates to palladium(II) chelates useful as diagnostic, therapeutic or theranostic agents, in particular for use in the diagnosis and/or treatment of proliferative diseases.

### BACKGROUND OF INVENTION

Palladium(II) is recognized as a cation of interest in medicine since the 1960s when its antitumor properties, similar to those of platinum(II), were explored. Nowadays, several Pd(II)-based drugs or therapeutic agents are used in more sophisticated techniques, such as targeted photodynamic therapy for localized cancer treatment. In nuclear medicine, palladium radioisotopes were first used in the early 1970s. ¹⁰⁹Pd-based molecules were described for the control of homograft rejection, and ¹⁰³Pd-metal was introduced in 1987 as implanted seeds for brachytherapy. Despite these early applications, further developments have remained very limited. However, these radiometals deserve special attention in therapy on account of their radiophysical characteristics, given the current needs for relevant radiopharmaceuticals. ¹⁰³Pd- and ¹⁰⁹Pd-radionuclides have half-lives (t_{1/2}) of 17 days and 13.7 hours, respectively. Palladium-103 decays by electron capture (EC) and Auger electron (AE) emissions to the ^{103m}Rh-daughter (t_{1/2} = 56 min), which is another AE emitter that decays to stable Rh. It is commercially available *via* cyclotron production with a high specific activity, no-carrier added product. ¹⁰⁹Pd-nuclide decays by β⁻ particle emission (E_{β(max)} = 1.12 MeV, 100% yield) to the daughter nuclide, silver-109m (t_{1/2} = 39.6 s), which in turn emits a photon (88 keV, 3.6% yield) accompanied by conversion electron emissions and AE emissions leading to the stable ¹⁰⁹Ag-nuclide. Palladium-109 is easily produced with decent specific activity from an isotopically enriched ¹⁰⁸Pd-target in a neutron reactor.

For the development of radiopharmaceuticals utilising metallic radionuclides, the radiometals produced in their cationic form must be complexed with a chelator that fulfills all the necessary criteria for *in vivo* applications. The complex formed with the radiometal should be very stable, both in terms of thermodynamic stability and kinetic inertness. Avantageously, the chelator could be bifunctional, *i.e.,* in addition to its coordination properties, it has at least one additional functional group suitable for conjugation to targeting biomolecules without compromising complex stability. The perfect match between the metallic cation and the design of the chelator is therefore the crucial factor for achieving the desired physico-chemical properties of the complex.

A particularly preferred application of therapeutic radiopharmaceuticals is their combination with a radiodiagnostic agent that allows for both patient disease staging and treatment monitoring ("theranostic"). Diagnostic radiotracers employ radionuclides, commonly metallic elements, with emissions that can be detected by specialised cameras and allow for disease imaging. These emissions include positrons (β⁺) and photons (γ) for PET (positron emission tomography) and SPECT (Single photon emission computed tomography), respectively. Radionuclides for therapy, such as lutetium-177 or yttrium-90, have been paired with gallium-68 for PET imaging, but the corresponding half-lives are not adequate (6.7 days for ¹⁷⁷Lu, 2.7 days for ⁹⁰Y *vs* 68 min for ⁶⁸Ga-nuclide) and their coordination properties differ considerably, with typical coordination numbers of 8-10 for Lu(III) and Y(III) *vs* 6 for Ga(III). In addition to these heterotopic pairs, a few pairs of homotopic elements, such as ⁶⁴Cu/⁶⁷Cu-isotopes, are of an increasing interest. The β⁺ emitter, copper-64 (t_{1/2}= 12.7 h) is produced worldwide for PET imaging in preclinical and clinical trials. The therapeutic partner, copper-67 (t_{1/2}= 61.8 h), is a β⁻ emitter but its rather low emission energy (E_{β-(max)} = 0.577 MeV) might limit its treatment potential. Finding alternative radionuclides with suitable therapeutic emissions to be paired with copper-64 would thus be highly beneficial.

While presenting interesting properties, palladium radioisotopes have been sparsely used in the context of radiopharmaceutical development, and only a few ligands such as DTPA and porphyrins were explored. One explanation for this is that the palladium coordination chemistry does not match the chelators considered as the "gold standards" in nuclear medicine, namely DOTA (1,4,7,10-tetraazacyclododecane-1,4,7,10-tetraacetic acid) and NOTA (1,4,7-triazacyclononane-1,4,7-triacetic acid) derivatives. Palladium, a second-row transition metal with the electronic configuration [Kr]4d¹⁰, mainly forms four-coordinate complexes with a square-planar geometry, which is not compatible with the structure of the previously cited ligands. Many ligands have been proposed for Pd(II) complexation, but examples providing highly stable and inert complexes are very rare. This is likely because many applications of Pd(II) chelates do not require such stringent properties *(e.g.,* as catalysts).

Cyclam is a well-known scaffold forming highly stable and inert complexes with small transition metal ions, which in particular forms a square-planar complex with Pd(II) with an extremely high stability constant (log *K* = 56.9), thus a very stable chelate (Harrington, J. M. et al., Inorganica Chimica Acta, September 2005, Vol. 358, pp. 4473-4480). However, this property has only been investigated for one other cyclam derivative, namely (tetra(2-hydroxyethyl) cyclam (Hay, R. W. et al., Journal of the Chemical Society, Dalton Transactions, 1987, pp. 2605-2613). The reason for this may be that there are notorious experimental difficulties in studying Pd(II) complexation in aqueous media, namely: the marked tendency of this metal cation to form insoluble hydrolysis species in all but the most acidic pH ranges, the frequently high stability of its polyamine complexes, and a predisposition to display slow complexation kinetics with structurally rigid ligands. An advantage of cyclam is that this molecule is easily functionalized with bioactive groups, typically in the central atom of the propylene bridge.

However, cyclam is not currently a good candidate for *in vivo* applications, in particular for radiotherapy, because no information whatsoever is available regarding (i) the inertness (resistance to dissociation) of a Pd(II) cyclam chelate and (ii) whether radiolabeling with radioisotopes of Pd(II) may be possible and how fast it would occur. Indeed, inertness is a mandatory requirement, because the chelate will be exposed to biological media comprising other cations, which may be able to replace the Pd(II) in the ligand. Such substitution would release the Pd(II) radionuclide within the body of the patient, thereby causing a loss of therapeutic efficacy and/or becoming a hazard to the patient's health. Inertness may be determined according to procedures known in the art, either in "cold" conditions (*i.e.,* with a nonradioactive cation) or in "hot" conditions (*i.e.,* with a radioactive cation).

Quantitative or near-quantitative radiolabeling yield (reflected in "labeling efficiency" or "LE") is another critical parameter for radiotherapeutic applications. relating to the possibility for and rate of complexation. Considering that radionuclides are involved, it is advantageous that the radiolabeling occurs as fast as possible. Mild complexation conditions (e.g., at room temperature) are naturally preferred over harsh ones *(e.g.,* at 90°C). To the Applicants' knowledge, cyclam was never used for radiolabeling with a Pd(II) radioisotope and thus, no information whatsoever was available about its Pd-labelling efficiency. It was however evidenced by the Applicants, as reported in the present application, that Pd(II) cyclam chelates do not fulfill all the necessary criteria for *in vivo* applications.

Thus, there is still a need for new Pd(II)-chelates with physicochemical properties compatible with radiotherapeutic applications. Moreover, there remains an interest in finding versatile chelating agents capable of complexing a theranostic pair, *i.e.,* two radionuclides suitable for diagnosis and therapy respectively.

In order to consistently investigate the effect of different N-substitutions of cyclam on the chelating properties over Pd(II) cations, the Applicants prepared the following series of cyclam derivatives shown on **Scheme 1** below. These derivatives were investigated for Pd-complexation

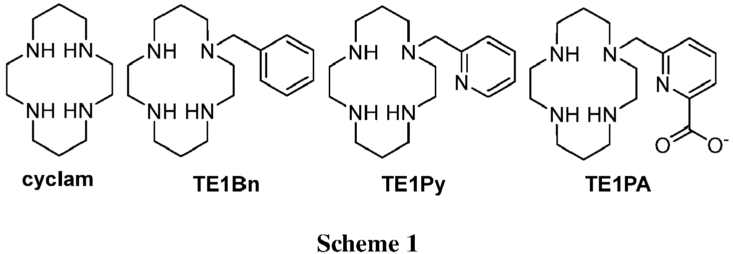

The Applicant surprisingly found out that, despite sharing the same cyclam core, the Pd(II)-chelates obtained from the ligands have significant differences in terms of chelate formation, chelate stability and suitability for radiolabeling. In particular, a large gap appears within the series between **TE1PA** and the other ligands. The presence of the "picolinate" pendant in **TE1PA** leads to outstanding coordination properties, thereby making **TE1PA** Pd(II)-chelates and its functionalized derivatives very attractive for radiotherapeutics.

### SUMMARY

The present invention relates to a chelate resulting from the complexation of a palladium(II) cation with a ligand of formula (I) 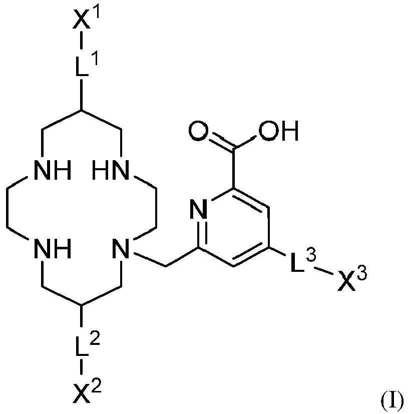 or a pharmaceutically acceptable salt and/or solvate thereof; wherein **L¹, L², L³, X¹, X²** and **X³** are as defined hereinafter.

According to one embodiment, the palladium(II) cation is a radioisotope of palladium(II). In one embodiment, the radioisotope of palladium(II) is selected from ¹⁰³Pd, ¹⁰⁹Pd, ¹⁰⁰Pd, ¹⁰¹Pd, ¹⁰⁷Pd, ^{111m}Pd and ¹¹²Pd; preferably selected from ¹⁰³Pd and ¹⁰⁹Pd. According to one embodiment, the ligand is 6-((1,4,8,11-tetraazacyclotetradecan-1-yl)methyl)picolinic acid (**HTE1PA**) or a pharmaceutically acceptable salt and/or solvate thereof.

The present invention further relates to a pharmaceutical composition comprising a chelate according to the invention and at least one pharmaceutically acceptable carrier. The present invention further relates to the chelate according to the invention or the pharmaceutical composition according to the invention for use as a medicament. The present invention further relates to the chelate according to the invention or the pharmaceutical composition according to the invention for use in a method of diagnosis *"in vivo"* and/or in the treatment of a proliferative disease. The present invention further relates to the use of a chelate according to the invention or a pharmaceutical composition according to the invention in biological imaging, preferably in medical imaging.

The present invention further relates to the use of a ligand as described herein in the manufacture of a chelate according to the invention. The present invention further relates to a process for manufacturing a chelate according to the invention, wherein the process comprises one of the following steps: (a1) a step of contacting the ligand with a stoichiometric amount of sodium tetrachloropalladate in refluxing water at a pH ranging from about 3 to about 5; (a2) a step of contacting a solution of the ligand with a solution of palladium(II) chloride in an aqueous medium at a temperature ranging from about 20°C to about 30°C, and at a pH ranging from about 0 to about 2.5; or (a3) a step of contacting a solution of the ligand with a solution of [¹⁰⁹Pd][PdCl₄]²⁻ at a temperature ranging from about 20°C to about 30°C, and at a pH ranging from about 2.5 to about 7.5.

### DEFINITIONS

In the present invention, the following terms have the following meanings.

### Chemical definitions

Where chemical substituents are combinations of chemical groups, the point of attachment of the substituent to the molecule is by the last chemical group recited on the right of the name of the substituent. For example, an arylalkyl substituent is linked to the rest of the molecule through the alkyl moiety and it may by represented as follows: "aryl-alkyl-". Unless otherwise indicated, the compounds were named using ChemDraw^{®} Professional 15.0 (PerkinElmer).

**"Activated carboxylic acid"** refers for example to acid anhydride or acyl halide.

**"Activated ester"** refers to an ester in which the alkoxy group is replaced by an electron-withdrawing group. Examples of activated esters are N-hydroxysuccinimide ester, N-hydroxyglutarimide ester, N-hydroxybenzotriazole ester, maleimide ester or pentafluorophenyl ester.

**"Aldehyde"** refers to a group comprising the connectivity C-C(=O)H, typically a -R-C(=O)H group, wherein R represents, for example, alkyl or aryl.

**"Alkene"** or **"alkenyl"** refer to any linear or branched hydrocarbon chain comprising at least 2 carbon atoms bound together by a double bond, typically comprising 2 to 16 carbon atoms, preferably 2 to 12 carbon atoms, more preferably 2 to 8 carbon atoms, furthermore preferably from 1 to 6 carbon atoms. Alkenyl groups may be monovalent or polyvalent (*e.g*., divalent). Examples of alkenyl groups are ethenyl, 2-propenyl, 2-butenyl, 3-butenyl, 2-pentenyl and its isomers, 2-hexenyl and its isomers, or 2,4-pentadienyl.

**"Alcohol"** refers to a group comprising the connectivity C-OH, typically a -R-OH group, wherein R represents, for example, alkyl or aryl.

**"Alkoxy"** refers to an alkyl-O- group.

**"Alkyl"** refers to a saturated linear or branched hydrocarbon chain, typically comprising from 1 to 16 carbon atoms, preferably from 1 to 12 carbon atoms, more preferably from 1 to 8 carbon atoms, furthermore preferably from 1 to 6 carbon atoms. Alkyl groups may be monovalent or polyvalent *(i.e.,* **"alkylene"** groups, which are divalent alkyl groups, are encompassed in "alkyl" definition). Although alkyl groups are generally linear, in the present application at least one **"cycloalkyl"** group may be included inside the structure of the main alkyl chain, for example a moiety having the structure alkyl-cycloalkyl-alkyl- (*e.g.*, *n*-butyl-cyclohexyl-ethyl- or ethyl-cyclopropylmethyl-) qualifies as an "alkyl" according to the present definition. Non-limiting examples of alkyl groups include methyl, ethyl, n-propyl, *i*-propyl, n-butyl, *i*-butyl, s-butyl and *t*-butyl, pentyl and its isomers *(e.g.,* n-pentyl, *iso*-pentyl), and hexyl and its isomers (*e.g., n*-hexyl, *iso*-hexyl). Preferred alkyl groups include methyl, ethyl, n-propyl, *i*-propyl, *n*-butyl, *s*-butyl and *t*-butyl (including methylene, ethylene, n-propylene, *n*-butylene and *n*-butylene).

**"Alkylaryl"** refers to an aryl group substituted by an alkyl group: alkyl-aryl-.

**"Alkylheteroaryl"** refers to an heteroaryl group substituted by an alkyl group: alkyl-heteroaryl-.

**"Alkyne"** or **"alkynyl"** refer to any linear or branched hydrocarbon chain comprising at least 2 carbon atoms bound together by a triple bond, typically comprising 2 to 16 carbon atoms, preferably 2 to 12 carbon atoms, more preferably 2 to 8 carbon atoms, furthermore preferably from 1 to 6 carbon atoms. Alkynyl groups may be monovalent or polyvalent (*e.g*., divalent). Examples of alkynyl groups are ethynyl, 2-propynyl, 2-butynyl, 3-butynyl, 2-pentynyl and its isomers, or 2-hexynyl and its isomers.

**"Amine"** refers to derivatives of ammonia (NH₃), wherein one or more hydrogen atoms have been replaced by a substituent such as, for example, alkyl or aryl. **"Amino"** refers to the -NH2 group.

**"Aryl"** refers to a cyclic, polyunsaturated, aromatic hydrocarbyl group comprising at least one aromatic ring and comprising from 5 to 12 carbon atoms, preferably from 6 to 10 carbon atoms. Aryl groups may be monovalent or polyvalent *(e.g.,* divalent). Aryl groups may have a single ring *(e.g.,* phenyl) or multiple aromatic rings fused together *(e.g.,* naphthyl) or linked covalently. The aromatic ring may optionally include one to two additional rings (either cycloalkyl, heterocycloalkyl or heteroaryl) fused thereto. This definition of "aryl" encompasses the partially hydrogenated derivatives of the carbocyclic systems enumerated herein, as long as at least one ring is aromatic. Non-limiting examples of aryl groups include phenyl, biphenyl, biphenylenyl, 5- or 6-tetralinyl, naphthalen-1- or -2-yl, 4-, 5-, 6 or 7-indenyl, 1- 2-, 3-, 4-or 5-acenaphthylenyl, 3-, 4- or 5-acenaphthenyl, 1- or 2-pentalenyl, 4- or 5-indanyl, 5-, 6-, 7- or 8-tetrahydronaphthyl, 1,2,3,4-tetrahydronaphthyl, 1,4-dihydronaphthyl, 1-, 2-, 3-, 4- or 5-pyrenyl. A particular example of aryl group is phenyl.

**"Carboxylate salt"** refers to an anion comprising at least one -COO⁻ group.

**"Carboxylate salt**" refers to a salt of a molecule, wherein the molecule comprises at least one carboxylic acid function (-COOH) and the salt is susceptible to be obtained by deprotonation of at least one carboxylic acid function of the molecule.

**"Coupling function"** refers to a function capable to react with another function to form a covalent linkage, *i.e.,* is covalently reactive under suitable reaction conditions, and generally represents a point of attachment for another substance. The coupling function is a moiety on a first compound that is capable of chemically reacting with a functional group on a second different compound to form a covalent linkage. Coupling functions generally include nucleophiles, electrophiles and photoactivatable groups. Non-limiting examples of coupling functions are amine (*e.g*., amino), isothiocyanate (-N=C=S), isocyanate (-N=C=O), activated ester (*e.g*., N-hydroxysuccinimide ester, N-hydroxyglutarimide ester or maleimide ester), carboxylic acid, activated carboxylic acid *(e.g.,* acid anhydride or acid halide), alcohol, alkene, *trans*-cyclooctene (TCO), alkyne, bicyclo[6.1.0]nonyne (BCN), dibenzocyclooctyne (DBCO), halide (*e.g*., Br or CI), azide (-N3), siloxy, phosphonic acid, thiol, tetrazine, norbornen, oxoamine, aminooxy (-O-NH2), thioether, haloacetamide, glutamate, glutaric anhydride, succinic anhydride, maleic anhydride, aldehyde, ketone, hydrazide, chloroformate (-C(=O)O-Cl) and maleimide.

**"Coupling product"** refers to the residue of a coupling function resulting from the reaction between two coupling functions, such as, for example, a functionally related group of atoms *(e.g.,* amide -C(O)-NH- group or a double bond) or a heterocycle *(e.g.,* a divalent triazolyl group). For example, reaction between two coupling functions A and B may lead to the following coupling products as shown on **Table 1** below, wherein **X** represents a halogen atom *(e.g.,* Br or CI).

**Table 1**

| **Coupling function A** | **Coupling function B** | **Coupling product** |
|---|---|---|
| R-N₃ | R-C=CH | Triazolyl (divalent) |
| R-NH₂ | R'-COOH | R-NH(C=O)-R' |
| R-SH | R'-SH | R-S-S-R' |
| R-OH | R'-(epoxide group) | R-OCH₂CH(OH)-R' |
| R-NH₂ | R'-(epoxide group) | R-NHCH₂CH(OH)-R' |
| R-SH | R'-(epoxide group) | R-SCH₂CH(OH)-R' |
| R-NH₂ | R'-CHO | R-N=CH-R' |
| R-NH₂ | R'-NCO | R-NH(C=O)NH-R |
| R-NH₂ | R'-NCS | R-NH(C=S)NH-R' |
| R-SH | R'-(C=O)CH₃ | R-(C=O)CH₂S-R' |
| R-SH | R'-O(C=O)X | R-S(C=O)O-R' |
| R-CH=CH₂ | R'-SH | R-CH₂CH₂S-R' |
| R-OH | R'-NCO | R-O(C=O)NH-R' |
| R-SH | R'-(C=O)CH₂X | R-SCH₂(C=O)-R' |
| R-NH₂ | R'-(C=O)N₃ | R-NH(C=O)-R' |
| R-COOH | R'-OH | R-C(=O)O-R' |
| R-COOH | R'-COOH | R-(C=O)O(C=O)-R' |
| R-OH | R'-X | R-O-R' |
| R-SH | R'-X | R-S-R' |
| R-NH₂ | R-CH₂C(NH²⁺)OCH₃ | R-NHC(NH²⁺)CH₂-R' |
| R-NHNH₂ | R'-CHO | R-NHNH=CH-R' |
| R-CH=CH-B(OH)₂ | R'-X | R-CH=CH-R' |
| R-C=CH | R'-X | R-C=C-R' |

**"Cycloalkyl"** refers to a cyclic alkyl group, typically comprising from 3 to 12 carbon atoms, preferably from 3 to 8 carbon atoms, more preferably from 3 to 6 carbon atoms. Cycloalkyl groups may be monovalent or polyvalent (*e.g*., divalent). This definition of "cycloalkyl" encompasses polycyclic cycloalkyls (*e.g*., bicycles) and bridged cycloalkyl structures.

"Cx-Cy" or "(Cx-Cy)" preceding the name of a group means that the group comprises from x to y carbon atoms, in accordance to common terminology in the chemistry field.

**"Halide"** refers to a fluorine, chlorine, bromine or iodine atom.

**"Haloacetamide"** refers to a -NH-C(O)CH₂X group, wherein X is a halide; *e.g.*, chloroacetamide, bromoacetamide or iodoacetamide.

**"Heteroalkyl"** refers to an alkyl group wherein one or more carbon atoms are replaced by a heteroatom selected from oxygen, nitrogen and sulfur, and wherein the resulting heteroalkyl group comprises at least one carbon atom. In heteroalkyl groups, the heteroatoms are bound along the alkyl chain only to carbon atoms, *i.e.,* each heteroatom is separated from any other heteroatom by at least one carbon atom, typically by at least two carbon atoms. Heteroalkyl groups may be monovalent or polyvalent (*e.g*., divalent). The nitrogen and sulfur heteroatoms may optionally be oxidized and the nitrogen heteroatoms may optionally be quaternized. Heteroalkyl groups may further include one or more =O and/or =S groups. Although heteroalkyl groups are generally linear, in the present application at least one **"heterocycloalkyl"** group may be included inside the structure of the main heteroalkyl chain, for example a moiety having the structure heteroalkyl-heterocycloalkyl-heteroalkyl- (*e.g.,* CH₃(O-CH₂CH₂)₂-(dioxanyl)-(CH₂CH₂-O)2CH₂- or CH3(NH-CH2CH2)2-(morpholinyl)-(CH2CH2-NH)2CH2-) qualifies as an "heteroalkyl" according to the present definition. In one embodiment, at least two carbon atoms are replaced by a heteroatom. In one embodiment, the heteroalkyl is bound to another group or molecule through a carbon atom, *i.e.,* the binding atom is not selected among the heteroatoms included therein. In one embodiment, the heteroalkyl is bound to another group or molecule through one of the heteroatoms included therein. Non-limiting examples of heteroalkyl include alkoxy, ethers and polyethers (*e.g*., polyethylene glycol), secondary and tertiary amines and polyamines, thioethers and polythioethers, and combinations thereof.

**"Heteroaryl"** refers to aromatic rings or aromatic ring systems comprising from 5 to 12 carbon atoms, preferably from 6 to 10 carbon atoms, having one or two rings which are fused together or linked covalently, wherein at least one ring is aromatic, and wherein one or more carbon atoms in one or more of these rings is replaced by oxygen, nitrogen and/or sulfur atoms. Heteroaryl groups may be monovalent or polyvalent *(e.g.,* divalent). The nitrogen and sulfur heteroatoms may optionally be oxidized and the nitrogen heteroatoms may optionally be quaternized. Non-limiting examples of heteroaryl groups include furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, tetrazinyl, imidazo[2,1-b][1,3]thiazolyl, thieno[3,2-b]furanyl, thieno[3,2-b]thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, isobenzofuranyl, benzothiophenyl, isobenzothiophenyl, indazolyl, benzimidazolyl, 1,3-benzoxazolyl, 1,2-benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1,2,3-benzothiadiazolyl, 2,1,3-benzothiadiazolyl, thienopyridinyl, purinyl, imidazo[1,2-a]pyridinyl, 6-oxopyridazin-1(6H)-yl, 2-oxopyridin-1(2H)-yl, 6-oxo-pyridazin-1(6H)-yl, 2-oxopyridin-1(2H)-yl, 1,3-benzodioxolyl, quinolinyl, isoquinolinyl, cinnolinyl, quinazolinyl and quinoxalinyl. Particular examples of heteroaryl groups are triazolyl, tetrazolyl, pyridinyl and tetrazinyl.

**"Heteroarylalkyl"** refers to an alkyl group substituted by an aryl group: heteroaryl-alkyl-.

**"Heterocycloalkyl"** refers to a cyclic heteroalkyl group, typically comprising from 2 to 11 carbon atoms, preferably from 2 to 7 carbon atoms, more preferably from 2 to 5 carbon atoms. Heterocycloalkyl groups may be monovalent or polyvalent *(e.g.,* divalent). Heterocycloalkyl groups are typically 3- to 7-membered, preferably 5- or 6-membered. Heterocycloalkyl are typically monocyclic or bicyclic, preferably monocyclic. This definition encompasses polycyclic heterocycloalkyls (*e.g.*, bicycles) and bridged heterocycloalkyl structures. Non-limiting examples of heterocycloalkyl include aziridine, pyrrolidine, piperidine, piperazine, morpholine, thiomorpholine, tetrahydrofuran and tetrahydropyran.

**"Hydrazide"** refers to a group comprising the connectivity C(=O)-NHNH₂, SO₂-NHNH₂ or (P=O)-NHNH₂, typically a -R(C=O)-NHNH₂, R-SO₂-NHNH₂ or RR'P(=O)-NHNH2 group, wherein R represents, for example, alkyl or aryl and R' represents, for example, hydrogen, alkyl or aryl.

**"Ketone"** refers to a group comprising the connectivity C-(C=O)-C, typically a -R-C(=O)-R' group, wherein R and R' each independently represents, for example, alkyl or aryl.

**"Linker"** refers to a single covalent bond or to a moiety comprising series of stable covalent bonds, the moiety often incorporating 1-40 plural valent atoms selected from the group consisting of C, N, O, S and P, that covalently attach a coupling function or a bioactive group as defined herein to a compound such as, for example, a ligand or chelate. The number of plural valent atoms in a linker may be, for example, 0, 1, 2, 3, 4, 5, 6, 7, 8, 9, 10, 20, 25 or 30. A linker may be linear or non-linear; some linkers may have pendant side chains or pendant functional groups (or both). Examples of such pendant moieties are water-solubilizing groups such as sulfo (-SO₃H or -SO₃⁻) or carboxylate (-COO⁻). In one embodiment, a linker is composed of any combination of single, double, triple or aromatic carbon-carbon bonds, carbon-nitrogen bonds, nitrogen-nitrogen bonds, carbon-oxygen bonds and carbon-sulfur bonds. Linkers may by way of example consist of a combination of moieties selected from alkyl, -C(O)NH-, -C(O)O-, -NH-, -S-, -O-, - C(O)-, -S(O)ₙ- where n is 0, 1 or 2; 5- or 6-membered monocyclic rings and optional pendant functional groups, for example sulfo, hydroxy or carboxy.
In the compounds of the invention, a coupling function may be reacted with a substance reactive therewith, whereby the linker becomes bonded to a bio-vectorizing group or to a water-solubilizing group. In this case, the linker may contain a coupling product resulting from the coupling reaction, such as, for example, the carbonyl group of an ester; the triazolo group resulting from a click reaction between an azide and an alkyne; or the -NHC(=S)NH- group resulting from the coupling of an amine on an isothiocyanate function.

**"Oxoamine"** refers to a group comprising the connectivity -NH₂-CH₂-C(=O)-, typically a -R-NH₂-CH₂-C(=O)-R' group, wherein R and R' interpedently represents, for example, alkyl or aryl.

**"Phosphonic acid"** refers to a -C-P(=O)(OR)₂ group, wherein each R interpedently represents, for example, hydrogen, alkyl or aryl.

**"Thioether"** or **"sulfide"** refers to a group comprising the connectivity C-S-C, typically a -S-R group, wherein the sulfur atom is bond to a carbon atom and wherein R represents, for example, alkyl or aryl.

**"Siloxy"** refers to a -O-Si(R)₃ group, wherein R represents, for example, alkyl or aryl.

**"Thiol"** refers to a group comprising the connectivity C-SH, typically a -R-SH group, wherein R represents, for example, alkyl or aryl.

### General definitions

**"About"** is used herein to mean approximately, roughly, around, or in the region of. The term "about" preceding a figure means plus or less 10 % of the value of the figure. When the term "about" is used in conjunction with a numerical range, it modifies that range by extending the boundaries above and below the numerical values set forth by 10%.

**"Administration"**, or a variant thereof (*e.g*., "**administering**"), means providing a therapeutic agent alone or as part of a pharmaceutically acceptable composition, to the patient in whom/which the condition, symptom, or disease is to be treated.

**"Antibody"** refers to monoclonal antibodies (mAb), polyclonal antibodies, multispecific antibodies (msAbs) (*e.g*., bispecific antibodies), hybrid or chimeric antibodies and antibody fragments, so long as they exhibit the desired biological activity. An "antibody fragment" comprises a portion of an intact antibody, preferably the antigen binding or variable region of the intact antibody. Examples of antibody fragments include Fab, Fab', F(ab')2, and Fv fragments; diabodies; linear antibodies; single-chain antibody molecules, especially single-chain variable fragment (scFv); and multispecific antibodies formed from antibody fragments.

**"Bioactive group"** refers to a molecule being able to recognize a predefined biological target (herein referred as **"bio-vectorizing group")** or to molecule for use in biological imaging (herein referred as **"bio-imaging group").** In particular, a bio-vectorizing group is selected to bind a specific biological target. In particular, a bio-vectorizing group is suitable to induce site-specific delivery of a compound *(e.g.,* a chelate) once administered to as subject. Typically, a "bioactive group" is selected from biomolecules, organic compounds and nanocarriers. "Biomolecules" include antibodies (*e.g*., polyclonal or monoclonal antibody, hybrid or chimeric antibody, single-domain antibody, dimeric or trimeric antibody fragment construct or minibody), peptides, proteins, lipids, monosaccharides, polysaccharides (including oligosaccharides), fatty acids, hapten, liposome, and polyamines (*e.g*., spermine). "Organic compounds" include fluorophores, chromophores and macrocyclic ligands. "Nanocarriers" include solid supports such as nanoparticles or polymeric microparticles, and cationic groups suitable for cellular internalization (*e.g*., liposomes). All examples of "bioactive groups" listed hereinabove typically qualifies as a "bio-vectorizing group", except for fluorophores and chromophores that typically qualifies as "bio-imaging group" only.

Bio-vectorizing groups and biological targets of interest are illustrated by the examples on **Table** 2 below.

**Table 2**

| **Group type** | **Biological target** | **Group family** | **Examples of bio-vectorizing group** |
|---|---|---|---|
| antibody | CD20 | anti CD20 | Tositumomab (BEXXAR), ibritumumab tiuxetan (Zevalin) Rituximab, Ofatumumab |
| antibody | CEA | anti CEA | IMMU-4, arcitumomab, M5A, T84, 2A3, 2A3-mFc, 9A6 |
| peptide | gastrin-releasing peptide (GRP) receptors | Bombesin, derivatives and analogs of bombesin | PEG4-Bombesin, Bombesin, - [D-Tyr6,βAla11,Thi13,Nle14]bom besin, PEG2-[D-Tyr6,βAla11,Thi13,Nle14]bom besin, -4-amino-1-carboxymethyl- piperidine- D-Phe-Gln- Trp-Ala- Val-Gly-His-Sta-Leu-NH2, D-Phe-Gln-Trp-Ala- Val-Gly- His-Sta-Leu-NH2, RGD-BBN |
| antibody | HER2 | anti HER2 | ZHER2:342, ZHER2:2891, ZHER2:2395, ZHER2:2891-ABD035 and derivatives thereof, ABY-025, ABY-028 and derivatives thereof |
| antibody | EGFR | anti EGFR | Cetuximab, panitumumab, L19-SIP |
| peptide | somatostatin receptors | somatostati n analogs | OCTREOTIDE, TATE, TOC, octreotate, 1-Nal3-octreotide (NOC), lanreotide , p-Cl-Phe-cyclo(D-Cys- Tyr- D-Aph(Cbm)- Lys- Thr-Cys )D-Tyr-NH2 (LM3), p-NO2-Phe-cyclo(D-Cys- Tyr- D-Aph(Cbm)- Lys- Thr-Cys )D-Tyr-NH2 (JR10), Cpa-cyclo(D-Cys- Tyr- D-Aph(Cbm)- Lys-Thr-Cys)D-Tyr-NH2, pansomatostatin |
| peptide | alphavbeta3 integrin | RGD peptides | Cyclo-RGD, RGD tetramer |
| minibody | PSMA - prostate | Anti-PSMA | HuJ591 minibody |
| mAb | PSMA - prostate | Anti-PSMA | J591, 7E11 |
| small molecule | carboxypepti dase of PSMA | urea-based inhibitors | Lys-urea-Asp sequence and derivatives thereof |
| small molecule | Bones | Bone mineralizati on | Phosphonates, biphosphonates |
| peptide | cholecystokin in-2 receptors (CCK) | CCK analogs | minigastrin |
| peptide | melanocortin - 1 receptor | α-MSH analogs | [Ser-Tyr-Ser-Met-Glu-His-Phe-Arg-Trp-Gly-Lys-Pro-Val]-NH2 |
| small molecules | melanocortin - 1 receptor | benzamide derivatives | benzamides derivatives |
| peptide | NK1-receptor glioblastoma | neuropeptid e | P substance |
| peptide | chemokine receptor 4 (CXCR4 ) | Chemokine analogs | fusine, CD 184, SDF-1a(CXCL12) |
| cationic group | mitochondria | - | Phosphonium moieties |

"**Biological imaging**" refers to any imaging method used in the technical field of biology, in particular medical imaging. Biological imaging may comprise a step of analysis of a biological sample by an imaging method *("in vitro* imaging" or *"ex vivo* imaging"). The biological sample is then obtained from a subject prior to the analysis step. Biological imaging may comprise a step of analysis of a subject, or of a part of the body of a subject, by a real time imaging method *("in vivo* imaging"). Biological imaging may be used in the diagnosis and/or for medical monitoring of the treatment of a disease *(e.g.,* a proliferative disease).

"**Chromophore"** or **"fluorophore**" refers to an organic chromophore (π-conjugated molecule) or an inorganic chromophore (metal complex). Non-limiting examples of organic chromophores are quinoline, coumarin, acridone, azathioxanthone, azaxanthone and phenanthridine. Non-limiting examples of inorganic chromophores are lanthanides π-conjugated complexes and ruthenium rhenium π-conjugated complexes.

**"Complex**" or "**chelate**" are synonyms and refer to the association of a ligand binding a metal ion. Chelation (or complexation) involves the formation or presence of two or more separate coordinate bonds between a polydentate (multiple bonded) molecule and a single central atom. Polydentate molecules are often organic compounds, and are called ligands, chelants, chelatants, chelators, chelating agents, or sequestering agents. Typically, the ligand is neutral or anionic and forms a chelate with a metallic cation.

**"Comprise"** or a variant thereof (*e.g*., "comprises", "comprising") is used herein according to common patent application drafting terminology. Hence, "comprise" preceded by an object and followed by a constituent means that the presence of a constituent in the object is required (typically as a component of a composition), but without excluding the presence of any further constituent(s) in the object. Moreover, any occurrence of "comprise" or a variant thereof herein also encompasses narrower expression "substantially consist of', further narrower expression "consist of' and any variants thereof (*e.g*., "consists of', "consisting of'), and may be replaced thereby, unless otherwise stated.

"**Hapten**" refers to a small molecule that can elicit an immune response only when attached to a large carrier such as, for example, a protein. Non-limiting examples of haptens are those disclosed in FR 2697255 A1 (Gruaz-Guyon et al.) and US 2012/0282178 A1 (McBride et al.)*,* especially the haptens listed in the claims thereof.

"**Human**" refers to a male or female human subject at any stage of development, including neonate, infant, juvenile, adolescent and adult.

"**Inertness**" refers to the resistance to dissociation of a chelate in given conditions. Preferably, the inertness corresponds to absence of dissociation. Conditions of particular interest are physiological conditions such as, for example, aqueous medium at 20-40°C and pH 6-8. Inertness may depend on whether the chelated cation is nonradioactive ("cold" inertness) or radioactive ("hot" inertness), for example due to differences in behavior of the radioactive cation, or because the conditions are different when handling and using radioactive material. In particular, dissociation can occur in the following cases: 1) competition with the formation of another chelate in presence of another ligand ("transchelation"); 2) competition with the chelation of another metal ("transmetalation") or any Lewis acid (the cation is then H⁺) ("acid-mediated dissociation"); or 3) oxidation/reduction of the metal cation.

**"Ligand"** or **"chelator"** or **"chelating agent"** are synonyms and refer to a polydentate molecule able to form coordination bonds with a metal ion to give a chelate. Preferably, the ligand is anionic.

**"Macrocyclic ligand"** refers to a cyclic molecule comprising at least 9 atoms among which at least 3 are heteroatoms such as, for example, O, S or N. Non-limiting examples of macrocyclic ligands are polyazamacrocycles, crown ethers and crown thioethers.

**"Patient"** refers to a subject who/which is awaiting the receipt of, or is receiving medical care or was/is/will be the object of a medical procedure, or is monitored for the development of the targeted disease or condition, such as, for example, a proliferative disease.

"**Peptide**" refers to a linear polymer of amino acids linked together by peptide bonds. Typically, a peptide comprises less than 50 amino acids. A peptide may be natural, synthetic or hemisynthetic. In one embodiment, the amino acids are natural amino acids.

"**Pharmaceutically acceptable**" means that the ingredients of a composition are compatible with each other and not deleterious to the subject to which/whom it is administered.

"**Pharmaceutically acceptable carrier**" refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by regulatory offices, such as, *e.g.,* FDA Office or EMA. Examples of pharmaceutically acceptable carriers include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (for example sodium carboxymethylcellulose), polyethylene glycol, polyacrylates, waxes, polyethylene- polyoxypropylene- block polymers, polyethylene glycol and wool fat.

**"Pharmaceutical composition"** refers to a composition comprising at least one therapeutic agent *(e.g.,* a chelate according to the present invention) and at least one pharmaceutically acceptable carrier.

**"Polysaccharide"** refers to a polymeric carbohydrate molecule composed of long chains of monosaccharide units bound together by glycosidic linkages, which may be linear or branched. Non-limiting examples include starch, glycogen, cellulose and chitin.

**"Prodrug"** refers to a pharmacologically acceptable derivative of a therapeutic agent *(e.g.,* a chelate of the invention) whose *in vivo* biotransformation product is the therapeutic agent (active drug). Prodrugs are typically characterized by increased bioavailability and are readily metabolized *in vivo* into the active compounds. Non-limiting examples of prodrugs include amide prodrugs and carboxylic acid ester prodrugs, in particular alkyl esters, cycloalkyl esters and aryl esters.

**"Protein"** refers to a macromolecule, typically a biomolecule, consisting of one or more peptides.

**"Selected from"** is used herein according to common patent application drafting terminology, to introduce a list of elements among which one or more item(s) is (are) selected. Any occurrence of "selected from" in the specification may be replaced by "selected from the group comprising or consisting of' and reciprocally without changing the meaning thereof.

**"Solvate"** refers to molecular complex comprising a compound along with stoichiometric or sub-stoichiometric amounts of one or more molecules of one or more solvents, typically the solvent is a pharmaceutically acceptable solvent such as, for example, ethanol. The term "**hydrate**" refers to a solvate when the solvent is water (H₂O).

"**Subject**" refers to an animal, typically a warm-blooded animal, preferably a mammal, more preferably a primate, furthermore preferably a human. In one embodiment, the subject is a "**patient**" as defined herein. In one embodiment, the subject is affected, preferably is diagnosed, with a proliferative disease. In one embodiment, the subject is at risk of developing a proliferative disease. Examples of risks factor include, but are not limited to, genetic predisposition, or familial history of proliferative diseases.

**"Therapeutic agent", "active pharmaceutical ingredient"** and **"active ingredient"** refer to a compound for therapeutic use and relating to health. Especially, a therapeutic agent *(e.g.,* a chelate according to the present invention) may be indicated for treating a disease *(e.g.,* a proliferative disease). An active ingredient may also be indicated for improving the therapeutic activity of another therapeutic agent.

**"Therapeutically effective amount"** (in short **"effective amount")** refers to the amount of a therapeutic agent *(e.g.,* a chelate according to the present invention) that is sufficient to achieve the desired therapeutic, prophylactic or preventative effect in the patient to which/whom it is administered, without causing significant negative or adverse side effects to the patient. A therapeutically effective amount may be administered prior to the onset of the disease for a prophylactic or preventive action. Alternatively, or additionally, the therapeutically effective amount may be administered after initiation of the disease for a therapeutic action.

**"Treating", "treatment"** or **"alleviation"** refers to both therapeutic treatment and prophylactic or preventative measures, wherein the object is to prevent or slow down (lessen) the targeted pathologic condition or disorder (herein a "**disease"**) *(e.g.,* a proliferative disease). Those in need of treatment include those already with the disease as well as those prone to have the disease or those in whom the condition or disease is to be prevented. A patient is successfully "treated" for a disease if, after receiving a therapeutic amount of a therapeutic agent *(e.g.,* a chelate according to the present invention), the patient shows observable and/or measurable reduction in or absence of one or more of the following: reduction in the number of pathogens; reduction in the percent of total cells that are pathogenic; and/or relief to some extent, one or more of the symptoms associated with the specific disease; reduced morbidity and mortality, and improvement in quality of life issues. The above parameters for assessing successful treatment and improvement in the disease are readily measurable by routine procedures familiar to a physician.

### DETAILED DESCRIPTION

### Chelate

An object of the present invention is a chelate resulting from the complexation of a palladium(II) cation with a ligand of formula (I) 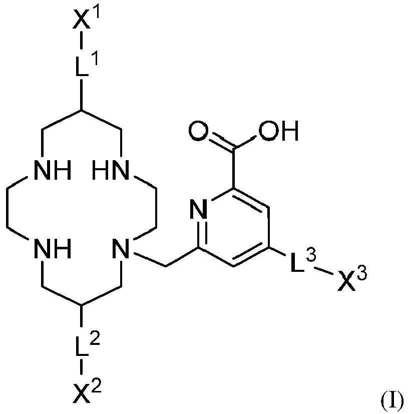 or a pharmaceutically acceptable salt and/or solvate thereof; wherein
**L¹, L²** and **L³** each independently represents:
   - a single bond; or
   - a linker selected from alkyl, arylalkyl, alkylaryl-alkyl, heteroalkylarylalkyl, alkenylaryl-alkyl, alkynylaryl-alkyl, heteroarylalkyl, alkylheteroaryl-alkyl, heteroalkylheteroaryl-alkyl, alkenylheteroarylalkyl, alkynylheteroaryl-alkyl, heteroalkyl, arylheteroalkyl, alkylarylheteroalkyl, heteroalkylaryl-heteroalkyl, alkenylaryl-heteroalkyl, alkynylaryl-heteroalkyl, heteroarylheteroalkyl, alkylheteroarylheteroalkyl, heteroalkylheteroaryl-heteroalkyl, alkenylheteroarylheteroalkyl, alkynylheteroaryl-heteroalkyl, aryl, alkylaryl, heteroalkylaryl, alkenylaryl, alkynylaryl, heteroaryl, alkylheteroaryl, heteroalkylheteroaryl, alkenylheteroaryl, alkynylheteroaryl, alkenyl, arylalkenyl, alkylaryl-alkenyl, heteroalkylaryl-alkenyl, alkenylarylalkenyl, alkynylaryl-alkenyl, heteroarylalkenyl, alkylheteroaryl-alkenyl, heteroalkylheteroaryl-alkenyl, alkenylheteroaryl-alkenyl, alkynylheteroaryl-alkenyl, alkynyl, arylalkynyl, alkylaryl-alkynyl, heteroalkylaryl-alkynyl, alkenylaryl-alkynyl, alkynylaryl-alkynyl, heteroarylalkynyl, alkylheteroaryl-alkynyl, heteroalkylheteroaryl-alkynyl, alkenylheteroaryl-alkynyl, alkynylheteroaryl-alkynyl,
      wherein the linker is optionally interrupted or terminated by at least one coupling product selected from triazolyl, NH(C=O), S-S, OCH₂CH(OH), NHCH₂CH(OH), SCH₂CH(OH), N=CH, NH(C=O)NH, NH(C=S)NH, (C=O)CH₂S, S(C=O)O, CH₂CH₂S, O(C=O)NH, SCH₂(C=O), NH(C=O), C(=O)O, (C=O)O(C=O), O, S, NHC(NH²⁺)CH₂, NHNH=CH, C=C and C=C;
X¹, X² and X³ each independently represents:
   - a hydrogen atom;
   - a coupling function selected from amine, isothiocyanate, isocyanate, activated ester, carboxylic acid, activated carboxylic acid, alcohol, alkene, trans-cyclooctene (TCO), alkyne, bicyclo[6.1.0]nonyne (BCN), dibenzocyclooctyne (DBCO), halide, azide, siloxy, phosphonic acid, thiol, tetrazine, norbornen, oxoamine, aminooxy, thioether, haloacetamide glutamate, glutaric anhydride, succinic anhydride, maleic anhydride, aldehyde, ketone, hydrazide, chloroformate, and maleimide; or
   - a bioactive group selected from antibody, peptide, protein, lipid, monosaccharide, polysaccharide, fatty acids, hapten, liposomes, polyamine, fluorophore, chromophore, macrocyclic ligand, nanoparticle, polymeric microparticle, cationic group suitable for cellular internalization, and a combination thereof.

According to one embodiment, the palladium(II) cation is a radioisotope of palladium(II). In one embodiment, the radioisotope of palladium(II) is selected from ¹⁰³Pd, ¹⁰⁹Pd, ¹⁰⁰Pd, ¹⁰¹ Pd, ¹⁰⁷Pd, ^{111m}Pd and ¹¹²Pd. In one particular embodiment, the radioisotope of palladium(II) is selected from ¹⁰³Pd(II) and ¹⁰⁹Pd(II). In one particular embodiment, the radioisotope of palladium(II) is ¹⁰³Pd(II). In one particular embodiment, the radioisotope of palladium(II) is ¹⁰⁹Pd(II).

According to one embodiment, **L**¹ and/or **L²** represents a linker selected from alkyl, arylalkyl and alkylaryl-alkyl; wherein the linker is optionally interrupted or terminated by at least one coupling product as defined herein. In one embodiment, **L¹** and/or **L²** represent a linker selected from -(CH₂)ₘ-, -phenyl-(CH₂)ₙ- or -(CH₂)ₚ-phenyl-(CH2)n-; wherein **m** is an integer ranging from 1 to 12, **n** is an integer ranging from 1 to 6 and **p** is an integer ranging from 1 to 6; wherein the linker is optionally interrupted or terminated by at least one coupling product as defined herein.

According to one embodiment, **X¹, X²** and/or **X³** represents a bioactive group as defined herein. In one embodiment, **X¹, X²** and/or **X³** represents a bio-vectorizing group selected from antibody, peptide, protein, lipid, monosaccharide, polysaccharide, fatty acids, hapten, liposomes, polyamine, macrocyclic ligand, nanoparticle, polymeric microparticle, cationic group suitable for cellular internalization, and a combination thereof. According to one embodiment, **X¹, X²** and/or **X³** represents a coupling function as defined herein. In one embodiment, at least one group selected from **X¹, X²** and **X³** represents a bioactive group as defined herein and at least one group selected from **X¹, X²** and **X³** represents a coupling function as defined herein.

According to one embodiment, -**L¹-X¹, -L²-X²** and/or **-L³-X³** represents a single bond and a hydrogen atom. In one embodiment, **-L¹-X¹** and/or **-L²-X²** represents a single bond and a hydrogen atom. In one particular embodiment, **-L²-X²** represents a single bound and a hydrogen atom.

All references herein to a chelate of the invention or a ligand as described herein include references to salts, solvates, multi component complexes and liquid crystals thereof. All references herein to a chelate of the invention or a ligand as described herein include references to polymorphs and crystal habits thereof. All references herein to a chelate of the invention or a ligand as described herein include references to isotopically-labelled compounds thereof, including deuterated compounds thereof. All references herein to a chelate of the invention or a ligand as described herein include references to stereoisomers thereof. All references herein to a chelate of the invention or a ligand as described herein include references to pharmaceutically acceptable prodrugs thereof.

In particular, the chelate of the invention or the ligand as described herein may be in the form of pharmaceutically acceptable salts. According to one embodiment, the chelate of the invention or the ligand as described herein is a pharmaceutically acceptable salt. Pharmaceutically acceptable salts include the acid addition and base salts thereof. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinafoate salts. Suitable base salts are formed from bases which form non-toxic salts. Examples include the aluminum, arginine, benzathine, calcium, choline, diethylamine, 2-(diethylamino)ethanol, diolamine, ethanolamine, glycine, 4-(2-hydroxyethyl)-morpholine, lysine, magnesium, meglumine, morpholine, olamine, potassium, sodium, tromethamine and zinc salts. Hemisalts of acids and bases may also be formed, for example, hemisulphate and hemicalcium salts. When a compound contains an acidic group as well as a basic group the compound may also form internal salts, and such compounds are within the scope of the invention. When a compound contains a hydrogen-donating heteroatom *(e.g.,* NH), the invention also encompasses salts and/or isomers formed by transfer of the hydrogen atom to a basic group or atom within the molecule. Pharmaceutically acceptable salts of compounds of the invention may be prepared by one or more of these methods: (i) by reacting the compound with the desired acid; (ii) by reacting the compound with the desired base; (iii) by removing an acid- or base-labile protecting group from a suitable precursor of the compound or by ring-opening a suitable cyclic precursor, *e.g.,* a lactone or lactam, using the desired acid; and/or (iv) by converting one salt of the compound to another by reaction with an appropriate acid or by means of a suitable ion exchange column. All these reactions are typically carried out in solution. The salt may precipitate from solution and be collected by filtration or may be recovered by evaporation of the solvent. The degree of ionization in the salt may vary from completely ionized to almost non-ionized.

According to one embodiment, the ligand is the form of an anion solubilized in a liquid such as, for example, an aqueous solution. In one embodiment, the anion is a carboxylate anion. In one particular embodiment, the anion is a carboxylate anion comprising exactly one -COO⁻ group. The anion *(e.g.,* a carboxylate anion) may be manufactured in the form of a salt from the corresponding acid *(e.g.,* a carboxylic acid) by using deprotonation methods well-known in the art such as, for example, the methods described hereinabove. The anion *(e.g.,* a carboxylate anion) may also be directly manufactured in the form of a salt such as, for example, by synthetizing the corresponding acid *(e.g.,* a carboxylic acid) in basic conditions.

According to one embodiment, the ligand as described herein is a pharmaceutically acceptable salt selected from hydrochloride/chloride and hydrobromide/bromide salts. According to one embodiment, the chelate of the invention is a pharmaceutically acceptable salt selected from hydrochloride/chloride and perchlorate salts.

In particular, the chelate of the invention or the ligand as described herein may be in the form of pharmaceutically acceptable solvates. According to one embodiment, the chelate of the invention or the ligand as described herein is a pharmaceutically acceptable solvate. According to one embodiment, the chelate of the invention or the ligand as described herein is a pharmaceutically acceptable salt and solvate.

In particular, the chelate of the invention or the ligand as described herein may include at least one asymmetric center(s) and thus may exist as different stereoisomeric forms. Accordingly, all references herein to a chelate of the invention or a ligand as described herein include all possible stereoisomers and include not only the racemic compounds, but the individual enantiomers and their non-racemic mixtures as well. Non-racemic mixtures may comprise any amounts of each distinct stereoisomer, for example one stereoisomer may be preponderant *(e.g.,* a 90/10 or 80/20 mixture), or the enantiomeric ratio may be close to a racemic mixture *(e.g.,* a 40/60 mixture). When a compound is desired as a single enantiomer, such single enantiomer may be obtained by stereospecific synthesis, by resolution of the final product or any convenient intermediate, or by chiral chromatographic methods as each are known in the art. Resolution of the final product, an intermediate, or a starting material may be carried out by any suitable method known in the art.

According to one embodiment, the ligand as described herein is selected from the compounds of **Table** 3 below, and pharmaceutically acceptable salts and/or solvates thereof.

**Table 3**

| Ref. | Formula | Name |
|---|---|---|
| **HTE1PA** | 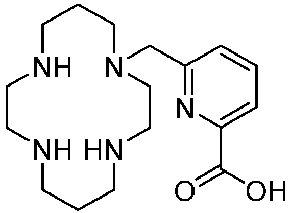 | 6-((1,4,8,11-tetraazacyclotetradecan-1-yl)methyl)picolinic acid |

According to one embodiment, the ligand is **HTE1PA,** *i.e.,* **L¹, L²** and **L³** each represents a simple bond, and **X¹, X**² and **X³** each represents hydrogen in formula (I).

In the present application, "**TE1PA"** refers to the 6-((1,4,8,11-tetraazacyclotetradecan-1-yl)methyl)picolinate of formula

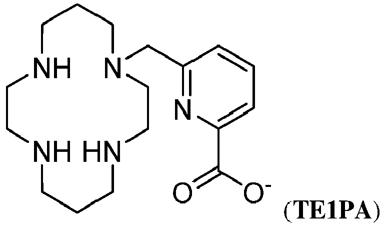

The **TE1PA** carboxylate anion may be manufactured from the **HTE1PA** carboxylic acid by using deprotonation methods well-known in the art such as, for example, the methods described hereinafter. The **TE1PA** carboxylate anion may also be directly manufactured such as, for example, by synthesizing the **HTE1PA** carboxylic acid in basic conditions.

According to one embodiment, the ligand is **TE1PA.** In one embodiment, the ligand is a **TE1PA** salt. In one particular embodiment, the ligand is a **TE1PA** pharmaceutically acceptable salt.

### Pharmaceutical composition

Another object of the present invention is a composition comprising a chelate of the invention, as described herein.

According to one embodiment, the composition further comprises at least one pharmaceutically acceptable carrier, so that the composition is a "pharmaceutical composition" as defined herein.

According to a first embodiment, the composition comprises the chelate of the invention as sole diagnostic agent and/or therapeutic agent. According to one embodiment, the composition comprises the chelate of the invention as sole diagnostic agent. According to one embodiment, the composition comprises the chelate of the invention as sole therapeutic agent.

According to a second embodiment, the composition further comprises at least another diagnostic agent and/or therapeutic agent. According to one embodiment, the other diagnostic agent is a radiodiagnostic agent. According to one embodiment, the other therapeutic agent is an antiproliferative agent.

In one embodiment, the radiodiagnostic agent is a copper(II) chelate. In one particular embodiment, the radiodiagnostic agent is a chelate resulting from the complexation of a copper(II) cation with a ligand as described in the present application such as, for example, a ligand of formula (I) herein or a pharmaceutically acceptable salt and/or solvate thereof. In one particular embodiment, the copper(II) cation is a copper(II) radioisotope. In one particular embodiment, the copper(II) radioisotope is selected from ⁶⁴Cu and ⁶⁷Cu.

Another object of the present invention is a medicament comprising a chelate of the invention, as described herein.

### Kit

Another object of the present invention is a kit of parts (in short "kit") comprising a chelate or composition of the invention, as described herein.

According to one embodiment, the kit comprises a manufacture such as, for example, a package or a container. According to one embodiment, the kit comprises instructions for use. The kit may be promoted, distributed, or sold as a unit for performing the methods of the present invention.

According to one embodiment, the kit comprises:
- a pharmaceutical composition comprising the chelate of the invention and
- another separate pharmaceutical composition comprising at least another diagnostic agent and/or therapeutic agent.

### Diagnostic use

Another object of the present invention is a chelate or a composition of the invention, as described herein, for use in a method of diagnosis *"in vivo"* of a disease.

According to one embodiment, the disease is a proliferative disease.

According to a first embodiment, the chelate or the composition is to be administered as sole diagnosis agent. According to a second embodiment, the chelate or the composition is to be administered before, concomitantly with or after at least another diagnostic agent. According to one embodiment, the other diagnostic agent is a radiodiagnostic agent such as, for example, the radiodiagnostic agent as described hereinabove.

### Medical use

Another object of the present invention is a chelate or a composition of the invention, as described herein, for use as a medicament.

Another object of the present invention is a chelate or a composition of the invention, as described herein, for use in the treatment of a proliferative disease.

Another object of the present invention is a method for treating a proliferative disease in a subject in need thereof, comprising administering to the subject a chelate or a composition of the invention, as described herein.

Another object of the present invention is the use of a chelate or a composition of the invention, as described herein, in the manufacture of a medicament for the treatment of a proliferative disease. Another object of the present invention is the use of a chelate or a composition of the invention, as described herein, for treating a proliferative disease.

According to one embodiment, the method or the use comprises a step of administering to a subject a therapeutically effective amount of a chelate, a composition or a medicament of the invention, as described herein.

According to one embodiment, the proliferative disease is a cancer such as, for example, leukemias, lymphomas and myelomas.

According to one embodiment, the composition or the medicament is to be is to be administered to a subject, and may be formulated using methods well-known in the art. Non-limiting examples of forms adapted for administration include solutions (such as, for example, sterile aqueous solutions), gels, dispersions, emulsions, suspensions and solid forms suitable for using to prepare solutions or suspensions upon the addition of a liquid prior to use (such as, for example, powder or liposomal forms).

The composition or the medicament may be administered using administration route well-known in the art such as, for example, parenterally, orally, by inhalation, spray, rectally, nasally, or via an implanted reservoir.

It will be however understood that the total daily usage of the chelate, the composition or the medicament will be decided by the attending physician within the scope of sound medical judgment. The specific therapeutically effective dose level for any particular patient will depend upon a variety of factors including the disease being treated and the severity of the disease; activity of the compound employed; the age, body weight, general health, sex and diet of the subject; the time of administration, route of administration, and rate of excretion of the specific therapeutic agent employed; the duration of the treatment; drugs used in combination or coincidental with the specific therapeutic agent employed; and like factors well known in the medical arts. For example, it is well within the skill of the art to start doses of the compound at levels lower than those required to achieve the desired therapeutic effect and to gradually increase the dosage until the desired effect is achieved. The total dose required for each treatment may be administered by multiple doses or in a single dose.

In one embodiment, the dosage of the chelate is about 0.01 to 500 mg per kg patient body weight per day, which can be administered in single or multiple doses. Preferably, the dosage level will be about 0.1 to about 250 mg/kg per day; more preferably about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be about 0.05 to 0.5, about 0.5 to 5 or about 5 to 50 mg/kg per day. The compounds may be administered on a regimen of 1 to 4 times per day, preferably once or twice per day. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

According to a first embodiment, the chelate, the composition or the medicament is to be administered as sole therapeutic agent.

According to a second embodiment, the chelate, the composition or the medicament is to be administered before, concomitantly with or after at least another therapeutic agent. According to one embodiment, the other therapeutic agent is an antiproliferative agent.

### Theranostic use

Another object of the present invention is a chelate, composition or medicament of the invention, as described herein, for use in a method of diagnosis *"in vivo"* and in the treatment of a disease. The disease object of the diagnosis and of the treatment is the same disease. According to one embodiment, the disease is a proliferative disease.

According to one embodiment, the chelate, the composition or the medicament is a theranostic agent. In one embodiment, the chelate, the composition or the medicament is a radiotheranostic agent.

According to one first embodiment, the diagnosis and the treatment are successive. According to another embodiment, the diagnosis and the treatment are concomitant.

According to a first embodiment, the chelate, the composition or the medicament is to be administered as sole diagnostic agent and/or therapeutic agent. According to one embodiment, the chelate, the composition or the medicament is to be administered as sole diagnostic agent. According to one embodiment, the chelate, the composition or the medicament is to be administered as sole therapeutic agent. According to a second embodiment, the chelate, the composition or the medicament is to be administered before, concomitantly with or after at least another diagnostic agent and/or therapeutic agent. According to one embodiment, the other diagnostic agent is a radiodiagnostic agent such as, for example, the radiodiagnostic agent as described hereinabove. According to one embodiment, the other therapeutic agent is an antiproliferative agent.

### Non-medical use

Another object of the present invention is the non-therapeutic use of a chelate or composition of the invention, as described herein, as an imaging agent.

According to one embodiment, the imaging agent is used in biological imaging. In one embodiment, the chelate is used in medical imaging. In one embodiment, the biological imaging is *"in vitro"* or *"ex vivo"* imaging. In one embodiment, the biological imaging concerns cancerous cells or tumors.

### Synthesis of the ligand

The ligand as described herein may be manufactured through synthetic methods well-known in the art.

According to one embodiment, the ligand is manufactured as described for **HTE1PA** ligand by Lima, L. M. P. et al. (Inorganic Chemistry, May 2012, Vol. 51, No. 12, pp. 6916-6927).

### Synthesis of the chelate

Another object of the present invention is the use of a ligand as described herein, in the manufacture of a chelate according to the present invention, as described herein.

Another object of the present invention is a process for manufacturing a chelate of the invention, as described herein, wherein the process comprises a step of contacting a ligand as described herein with a palladium(II) cation.

According to one embodiment, the palladium(II) cation is in the form of a palladium(II) salt. In one embodiment, the palladium(II) salt is an inorganic salt. In one particular embodiment, the palladium(II) salt is sodium tetrachloropalladate (Na₂PdCl₄) or palladium(II) chloride (PdCl₂).

According to one embodiment, the palladium(II) cation is present in a liquid. According to one embodiment, the ligand is present in a liquid. In one embodiment, the liquid is an aqueous medium. In one embodiment, the liquid is a solution.

According to one embodiment, the process comprises a step of contacting the ligand with a stoichiometric amount of sodium tetrachloropalladate (Na₂PdCl₄) in refluxing water at a pH ranging from about 3 to about 5. In one embodiment, the pH is about 4. This embodiment is especially relevant for solid-state studies of the chelate.

According to one embodiment, the process comprises a step of contacting a solution of the ligand with a solution of palladium(II) chloride (PdCl₂) in an aqueous medium at a temperature ranging from about 20°C to about 30°C, and at a pH ranging from about 0 to about 2.5. In one embodiment, the temperature is about 25°C. In one embodiment, the pH ranges from about 0 to about 1.5. In one embodiment, the solution comprises an electrolyte such as, for example, potassium chloride (KCl) *(e.g.,* at a concentration of about 1.0 M). This embodiment is especially relevant for carrying out thermodynamic studies.

According to one embodiment, the process comprises a step of contacting a solution of the ligand with a solution of [¹⁰⁹Pd][PdCl₄]²⁻ at a temperature ranging from about 20°C to about 30°C, and at a pH ranging from about 2.5 to about 7.5; preferably from about 2.5 to about 4.5 or from about 7 to about 7.5. In one embodiment, the temperature is about 25°C. In one embodiment, the pH is about 3.5. In one embodiment, the solution comprises a buffer such as, for example, ammonium acetate (NH₄OAc) (*e.g.,* at a concentration of about 0.1 M). This embodiment is especially relevant for carrying out radiolabeling assays.

The pH may be adjusted to the desired value by methods well-known in the art such as, for example, the use of a buffer solution or addition of a solution comprising an acid *(e.g.,* HCl) or a base *(e.g.,* NaOH).

### BRIEF DESCRIPTION OF THE DRAWINGS

**Figure 1** is a diagram showing the ORTEP diagram of [Pd(**cyclam**)]²⁺ cation plotted at 30% of ellipsoids probability.
**Figure 2** is a diagram showing the ORTEP diagram of the crystal structure of [Pd(**TE1Bn**)]²⁺ cation plotted at 30% of ellipsoids probability.
**Figure 3** is a diagram showing the ORTEP diagram of [Pd(**TE1Py**)]²⁺ cation plotted at 30% of ellipsoids probability.
**Figure 4** is a diagram showing the ORTEP diagram of the crystal structure of [Pd(**HTE1PA**)]²⁺ obtained from a 5 M HCl solution.
**Figure 5** is a diagram showing the ORTEP diagram of the crystal structure of [Pd(**HTE1PA**)]²⁺ at pH 3.5.
**Figure 6** is a diagram showing the ORTEP diagram of the crystal structure of [Pd(**TE1PA**)]⁺ at pH 7, plotted at 30% of ellipsoids probability.
**Figure 7** is a graph showing ¹H NMR spectra of acid-assisted dissociation of the [Pd(**cyclam**)]²⁺ in 5 M DC1 at 90°C, at t = 0 (bottom) and t = 4 months (top).
**Figure 8** is a graph showing the ¹H NMR spectra of acid-assisted dissociation of the [Pd(**TE1PA)**]²⁺ in 5 M DCl at 90°C, at t = 0 (bottom) and t = 5 months (top).
**Figure 9** is a graph showing the % labeling efficiency for ¹⁰⁹Pd-radiolabeling reactions of the macrocyclic ligands at pH 3.5, 7 and 8.5 in 0.1 M NH₄OAc at 90°C for 30 minutes.
**Figure 10** is a graph showing the % labeling efficiency for ¹⁰⁹Pd-radiolabeling reactions of the macrocyclic ligands at pH 3.5, 7 and 8.5 in 0.1M NH₄OAc at 90°C for 10 minutes.
**Figure 11** is a graph showing the % labeling efficiency for ¹⁰⁹Pd-radiolabeling reactions of the macrocyclic ligands at pH 3.5, 7 and 8.5 in 0.1 M NH₄OAc at 25°C for 10 minutes.
**Figure 12** is a histogram showing the comparison of ¹⁰⁹Pd-labeling of selected macrocyclic ligands at pH 3.5 in 0.1 M NH₄OAc after 10 minutes at room temperature (25 °C) and 90 °C.
**Figure 13** is a graph showing the EDTA challenge (1000 eq) of [¹⁰⁹Pd][Pd(**TE1PA**)]⁺ and [¹⁰⁹Pd][Pd(**cyclam**)]²⁺ in PBS (0.01 M, pH 7) at 25 °C over a 24 h period.

### EXAMPLES

The present invention is further illustrated by the following examples.

### Example 1: Synthesis of the compounds

### Material and methods

Reagents were purchased from Acros Organics^{®} or Sigma-Aldrich^{®} and used without further purification. Ultrapure water was freshly obtained from a Milli-Q dispenser. A solvent purification system was used to dispense dry solvents before each reaction. X-ray diffraction and NMR spectra were recorded at the "Services communs" of the University of Brest. ¹H and ¹³C NMR spectra were recorded using Bruker Avance 500 (500 MHz), Bruker Avance 400 (400 MHz) or Bruker AMX-3 300 (300 MHz) spectrometers. Coupling constants are given in hertz and chemical shifts in ppm.

Mass spectrometry: The high-resolution mass spectra of compounds were recorded at Institute of Organic and Analytic Chemistry (ICOA, Orleans, France) with a HRMS Q-Tof MaXis instrument, using ESI, APCI, APPI or nano-ESI for ionization. The low-resolution Mass Spectrometry analyses were performed at the "Services communs" of the University of Brest on a Waters Synapt XS, source ESI.

X-ray diffraction: Single-crystal X-ray diffraction data were collected with a Xcalibur 2 CCD 4-circle Diffractometer (Oxford Diffraction) fitted with a graphite monochromated Mo Kα radiation (λ = 0.71073 Å). Data were collected at 170 K. Unit cell determination and data reduction, including interframe scaling, Lorentzian, polarization, empirical absorption, and detector sensitivity corrections, were carried out using attached programs of CrysAlis software (Oxford Diffraction). Structures were solved by direct methods and refined by full matrix least-squares method on F 2 with the SHELXL97 suite of programs. The hydrogen atoms were identified at the last step and refined under geometrical restraints and isotropic U-constraints. **CCDC 2159598-2159602** contain the supplementary crystallographic data. These data can be obtained free of charge from The Cambridge Crystallographic Data Centre via www.ccdc.cam.ac.uk/data_request/cif.

### Synthesis of the ligands

### Material and methods

**Cyclam** was purchased from CheMatech. **TElBn** was synthesized as previously described (Le Baccon, M. et al., New Journal of Chemistry, August 2001, Vol. 25, pp. 1168-1174) **TE1PA** was synthesized as previously described (Lima, L. M. P. et al., Inorganic Chemistry, May 2012, Vol. 51, No. 12, pp. 6916-6927).

**TE1Py** was synthesized as described on **Scheme 2** below. 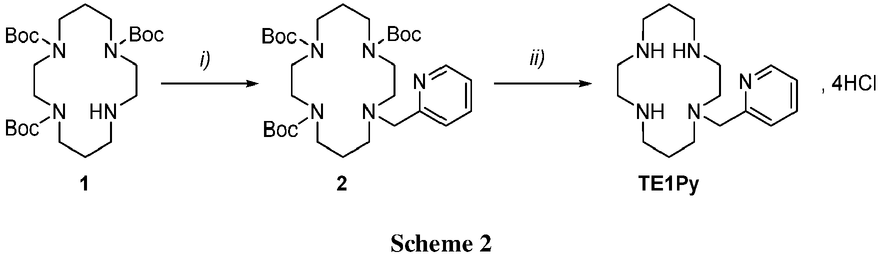 Synthesis of **TE1Py4HCl.** Reagents and conditions: i) 2-(Chloromethyl)pyridine hydrochloride, NaI, K₂CO₃, CH₃CN, reflux, Ar, 4 days; *ii)* 3M HCl, reflux, 6h.

Starting compound **1,4,8,11-Tetraazacyclotetradecane-1,4,8-tricarboxylic acid tri-tert-butyl ester (1)** was prepared as previously described by Herrero, C. et al. (Physical Chemistry Chemical Physics, February 2014, Vol. 16, pp. 12067-12072).

### Synthesis of ligand TE1Py

**Compound (2).** 2-(Chloromethyl)pyridine hydrochloride (161 mg, 0.982 mmol) was dissolved in 5 mL of 2 M K₂CO₃ aqueous solution. The solution was stirred for 15 min at room temperature and then extracted with Et₂O (3×3 mL). The organic phase was dried over MgSO₄, filtered and evaporated under vacuum at 25°C. The resulting 2-(chloromethyl)pyridine as a red liquid (97 mg, 0.760 mmol, 1.1 eq) was then dissolved in dry CH₃CN (5 mL) and added to a solution of tri-Boc cyclam **1** (346 mg, 0.691 mmol, 1 eq) in dry CH₃CN (10 mL). NaI (5 mg, 0.035 mmol, 0.05 eq) and K₂CO₃ (764 mg, 5.528 mmol, 8 eq) were added and the solution was stirred at 80°C for 2.5 days. The solution was cooled down to room temperature and filtered off to remove K₂CO₃ salts. After evaporation of the filtrate, the residue was dissolved in CHCl₃ and the solution was filtered again to remove the K₂CO₃. The crude product was purified by chromatographic silica column eluted with CH₂Cl₂/AcOEt (100:0 to 60:40) to give compound 2 as a white foam (352 mg, 78%). ¹H NMR (CD3CN, 500 MHz, 70°C) δ (ppm): 8.47 (d, *³J* = 5.0 Hz, 1H, C*H*_{Ar} py), 7.67 (t, *³J* = 7.9 Hz, 1H, C*H*_{Ar} py), 7.38 (d, *³J* = 7.9 Hz, 1H, C*H*_{Ar} py), 7.18 (t, *³J* = 5.0 Hz, 1H, C*H*_{Ar} py), 3.69 (s, 2H, C*H₂* py), 3.38-3.21 (m, 12H, C*H*₂α-N), 2.72-2.66 (m, 2H, C*H*₂α-N), 2.52-2.45 (m, 2H, C*H*₂α-N), 1.92-1.85 (m, 2H, C*H*₂β-N), 1.74-1.67 (m, 2H, C*H*₂β-N). ¹³C NMR (CD3CN, 125 MHz, 70°C) *δ* (ppm): 160.7 (Cipso py), [156.7,156.6,156.5] (*C*=O Boc), [150.0,137.2,124.6,123.1] (CHArpy), [80.1, 80.079.9] (*C*_{quat} Boc), 62.1 (*C*H₂ py), [55.1,55.3,48.5 ( × 2), 48.3, 48.2, 47.9, 47.0] (*C*H₂α-N), [29.9, 28.1] (*C*H₂β-N), [29.1 ( × 2), 29.0] (*C*H₃ Boc). HR-MS (ESI⁺): *m*/*z* calcd. for [C₃₁H₅₄N₅O₆]⁺ 592.4069, found 592.4075, [M+H]⁺; calcd. for [C₃₁H5₃N₅NaO₆]⁺ 614.3888, found 614.3887, [M+Na]⁺.

**TE1Py 4HCl.** Cyclam derivative **2** (352 mg, 0.595 mmol) was dissolved in 3M HCl (10 mL) and the reaction was stirred at reflux for 6 h. The mixture was concentrated, followed by the addition of excess of acetone. The precipitate was filtered to yield compound **TE1Py** 4HCl as a white powder (242 mg, 93%). ¹H NMR (400 MHz, CDCl₃, 25°C) δ (ppm): 8.73 (d, *³J* = 6.0 Hz, 1H, C*H*_{Ar} py), 8.54 (t, *³J* = 8.0 Hz, 1H, C*H*_{Ar} py), 8.04 (d, *³J* = 8.0 Hz, 1H, C*H*_{Ar} py), 7.97 (dd, *³J* = 6.0 Hz, 8.0 Hz , 1H, C*H*_{Ar}py), 4.12 (s, 2H), 3.60-3.52 (m, 4H, C*H*₂α-N), 3.45-3.40 (m, 2H, C*H*₂α-N), 3.39-3.34 (m, 2H, C*H*₂α-N), 3.33-3.27 (m, 2H, C*H*₂α-N), 3.26-3.20 (m, 2H, C*H*₂α-N), 2.98-2.93 (m, 2H, C*H*₂α-N), 2.70-2.65 (m, 2H, C*H*₂α-N), 2.33-2.25 (m, 2H, C*H*₂β-N), 2.01-1.92 (m, 2H, C*H₂*β-N). ¹³C NMR (D₂O, 500 MHz, 25°C) *δ* (ppm): 154.6 (*C*ᵢₚₛₒ py), [150.3, 144.3, 130.7, 129.3] (*C*H_{Ar} py), 58.0 (*C*H₂ py), [53.1, 50.9, 45.7, 45.4, 44.9, 44.4, 41.9, 41.5] (*C*H₂α-N), [24.8, 22.3] (*C*H₂β-N). HR-MS (ESI⁺): *m*/*z* calcd. for [C₁₆H₃₀N₅]⁺ 292.2496, found 292.2495, [M+H]⁺; calcd. for [C₁₆H₃₁N₅]²⁺ 146.6284, found 146.6285, [M+2H]²⁺.

### Synthesis of the chelates

### General procedure for the synthesis of Pd(II) complexes

The ligand (1 eq) was dissolved in water (C = 0.06 M) and the pH of the solution was adjusted to 4 before the addition of 1.4 eq of Na₂PdCl₄ in water (C = 0.08 M). The reaction was heated to reflux and the pH was adjusted again to 4 by addition of NaOH solution. The mixture was then stirred overnight under reflux. After cooling to room temperature, solid impurities were filtered off and the solution was evaporated under vacuum. The residue was dissolved in a minimal amount of dry methanol. The insoluble salts formed were filtered off and the filtrate was evaporated to dryness. This procedure was repeated until there were no more salts.

### Results

**Palladium(II) 1,4,8,11-tetraazacyclotetradecane dichloride: [Pd(cyclam)](Cl)₂.** Yellowish powder, 72 mg, yield 63%. ¹H NMR (500 MHz, D₂O, 25°C, pD = 7.0) *δ* (ppm): 3.08-2.85 (m, 10H, C*H*₂α-N), 2-73-2-57 (m, 6H, C*H*₂α-N), 2.18-2.10 (m, 2H, C*H*₂β-N), 1.62-1.50 (m, 2H, C*H₂*β-N) [major: *trans-III* and minor: *trans*-I; each series were not identified separately due to the complexity/superposition of the signals]. ¹³C NMR (500 MHz, D₂O, 25°C, pD = 7.0) *δ* (ppm): [57.4^{M}, 56.7, 54.8^{M}, 54.1] (CH₂α-N), [32.6^{M}, 32.4] (*C*H₂β-N) [M = major series: *trans-III,* minor series: *trans*-I]*.* HR-MS (ESI⁺): *m*/*z* calcd. for [C₁₀H₂₄N₄Pd]²⁺ 153.0512, found 153.0520, [M]²⁺; calcd. for [C₁₀H₂₃N₄Pd]⁺ 305.0952, found 305.0955, [M-H]⁺.

**Palladium(II) benzyl-1,4,8,11-tetraazacyclotetradecane tetrachloropalladate: [Pd(TE1Bn)](PdCl₄).** This complex was synthesized using the same procedure described for the other complexes but after stirring the solution overnight under reflux, the complex precipitated. After cooling to room temperature, the complex was filtered off. Brown powder, 23 mg, yield = 51%, mixture of configurations. ¹H NMR (500 MHz, (CD₃)₂SO, 25°C) *δ* (ppm): 7.66-7.60 (m, 2H, C*H*_{Ar} Bn), 7.52-7.44 (m, 3H, C*H*_{Ar} Bn), 4.71 (d, *²J* = 13.7 Hz, 1H, C*H*₂ Bn), 4.20 (d, *²J* = 13.7 Hz, 1H, C*H*₂ Bn), 3.04-2.16 (m, 20H, C*H*₂α-N, C*H*₂β-N), 2.07-1.89 (m, 2H, C*H*₂α-N, C*H*₂β-N), 1.51-1.37 (m, 1H, C*H*₂β-N) [major series = *trans*-I]*.* ¹³C NMR (125 MHz, (CD₃)₂SO, 25°C) *δ* (ppm): 131.8 (*C*H_{Ar} Bn) , 130.7 (*C*ᵢₚₛₒ Bn), 129.2 (*C*H_{Ar} Bn), [59.3, 58.2, 56.2, 54.2, 52.7, 51.4, 50.7, 50.4, 49.9] (*C*H₂α-N, *C*H₂ Bn), [28.1, 25.3] (*C*H₂β-N) [major series = *trans*-I]. HR-MS (ESI⁺): *m*/*z* calcd. for [C₁₇H₃₀N₄Pd]²⁺ 198.0747, found 198.0760, [M]²⁺; calcd. for [C₁₇H₂₉N₄Pd]⁺ 395.1422, found 395.1433, [M-H]⁺; calcd. for [C₁₇H₃₀ClN₄Pd]⁺431.1188, found 431.1199, [M+Cl]⁺.

**Palladium(II) 2-pyridylmethyl-1,4,8,11-tetraazacyclotetradecane tetrachloropalladate: Pd(TE1Py)](PdCl₄).** Yellow powder, 19 mg, yield 58%. ¹H NMR (500 MHz, D₂O,pD = 7, 25°C) *δ* (ppm): 8.78 (d, *³J* = 4.4 Hz, 1H, C*H*_{Ar}py), 8.00 (t, *³J* = 7.7 Hz, 1H, C*H*_{Ar} py), 7.74 (d, *³J* = 7.7 Hz 1H, C*H*_{Ar} py), 7.59 (dd, *³J* = 7.7 Hz, 4.4 Hz, 1H, C*H*_{Ar} py), 4.64 (d, *²J* = 14.0 Hz, 1H, *C*H₂ py), 4.04 (d, *²J* = 14.0 Hz, 1H, C*H*₂ py), 3.23-2.57 (m, 14H, C*H*₂α-N), 2.46-2.02 (m, 5H, C*H*₂α-N, C*H*₂β-N), 1.59-1.44 (m, 1H, C*H₂*β-N) [major series = *trans-III].* ¹³C NMR (125 MHz, D₂O, 25°C, pD = 7.0) *δ* (ppm): 154.6 (*C*ᵢₚₛₒ py), [152.7, 141.4, 129.5, 127.7] (*C*H_{Ar} py), [65.2, 62.9, 62.8, 56.9, 56.2, 54.2 (X2), 53.7, 53.3] (*C*H₂α-N, *C*H₂ py), [30.9, 28.9] (*C*H₂β-N) [major series = *trans*-III]. HR-MS (ESI⁺): *m*/*z* calcd. for [C₁₆H₂₉N₅Pd]²⁺ 198.5723, found 198.5732, [M]²⁺; calcd. for [C₁₆H₂₈N₅Pd]⁺ 396.1374, found 396.1384, [M-H]+; calcd. for [C₁₆H₂₉ClN₅Pd]⁺ 432.1141, found 432.1147, [M+Cl]⁺.

**Palladium (II) 6-methylpicolinate-1,4,8,11-tetraazacyclotetradecane tetrachloropalladate: [Pd(TE1PA)](PdCl₄).** Yellow powder, 26 mg, yield 65%. ¹H NMR (500 MHz, D₂O,pD=7, 25°C) *δ* (ppm): 8.10-7.98 (m, 5H, C*H*_{Ar}pico), 7.79 (d, *³J* = 7.0 Hz, 1H, C*H*_{Ar}pico)*,* 7.58 (d, *³J* = 6.4 Hz, 1H, C*H*_{Ar}pico), 5.99-5.89 (m, 1H, *NH),* 5.74-5.65 (m, 1H, NH), 5.31-5.18 (m, 2H, NH), 4.56-4.45 (m, 3H, C*H*₂ pico), 3.86 (d, *²J* = 13.9 Hz, 1H, C*H*₂ pico), 3.83-3.73 (m, 3H, C*H*₂ pico, C*H*₂α-N), 3.56-3.46 (m, 2H, C*H*₂α-N), 3.40-2.51 (m, 41H, C*H*₂α-N), 2.43-2.02 (m, 10H, C*H*₂α-N, C*H*₂β-N), 1.96-1.89 (m, 1H, C*H*₂β-N), 1.57-1.36 (m, 3H, C*H*₂α-N, C*H₂*β-N) [major: *trans-III* and minor: *trans*-I; each series was not identified separately due to the complexity/superposition of the signals)]. ¹³C NMR (125 MHz, D₂O, 25°C, pD = 7.0) *δ* (ppm): [173.3^{M}, 172.8] (*C*=O pico), [154.3, 153.9, 153.3, 153.2] (*C*ᵢₚₛₒ pico), [139.4, 138.6^{M}, 127.3, 126.1^{M}, 123.9, 123.3^{M}] (*C*H_{Ar} pico), [65.7, 63.3, 62.2, 60.1^{M}, 59.9^{M}, 58.1^{M}, 53.8, 53.6, 53.2^{M}, 52.7^{M}, 52.1, 51.8^{M}, 51.3, 50.8, 50.4, 50.1^{M}, 50.0^{M}, 49.5^{M}] (*C*H₂α-N, *C*H₂ pico), [28.8^{M}, 27.9, 26.5, 24.7^{M}] (*C*H₂β-N) [M = major series: *trans-III,* minor series: *trans*-I]*.* HR-MS (ESI⁺): *m*/*z* calcd fo [C₁₇H₂₉N₅O₂Pd]²⁺ 220.5673, found 220.5680; *m*/*z* calcd for [C₁₇H₂₈N₅O₂Pd]⁺ 440.1259, found 440.1274.

### X-ray structures

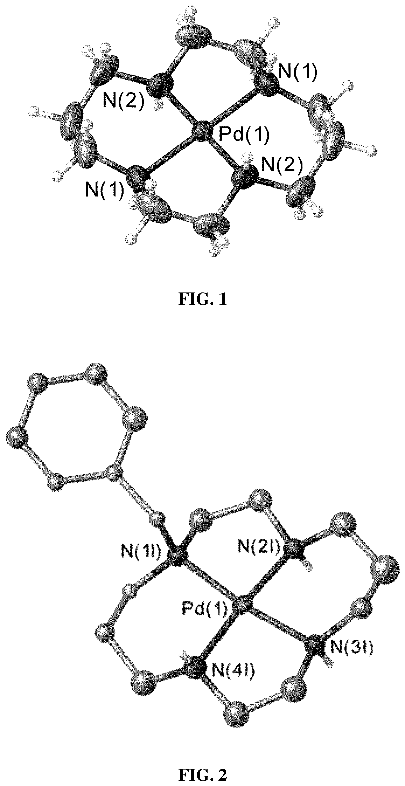

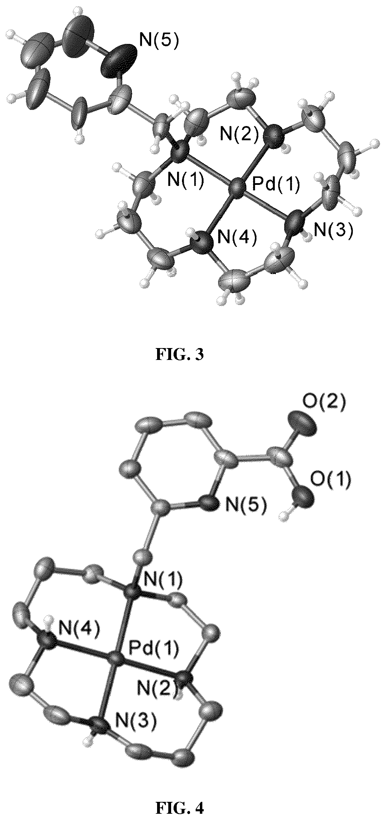

The structures of Pd(**cyclam)**]²⁺, **[Pd(TE1Bn**)]²⁺ and Pd(**TE1Py)**]²⁺ are shown respectively in **Figure 1**, **Figure 2** and **Figure 3**. The structures of [Pd(**HTE1PA**)]²⁺ and [Pd(**TE1PA**)]⁺ obtained by X-ray diffraction are shown in **Figures 4-6**. Selected bond lengths and angles for all complexes are given in **Table 4** below.

**Table 4**

| | [Pd(**cylam)**]²⁺ | [Pd(**TE1Bn**)]²⁺ | [Pd(**TE1Py**)]²⁺ |
|---|---|---|---|
| Config. | \| *trans*-III | *trans*-III | *trans*-III |
| Pd(1)-N(1) | 2.039(6) | 2.012(19) | 2.074 |
| Pd(1)-N(2) | 2.031(5) | 1.978(15) | 2.024 |
| Pd(1)-N(3) | - | 2.036(16) | 2.042 |
| Pd(1)-N(4) | - | 1.997(16) | 2.040 |
| Pd(1)-N(5) | - | - | - |
| N(2)-H(2)---O(1) | - | - | - |
| N(1)-Pd(1)-N(2) | 85.3(3) | 86.0(7) | 84.33 |
| N(2)-Pd(1)-N(3) | 94.7(3) | 86.8(6) | 94.54 |
| N(3)-Pd(1)-N(4) | - | 89.4(7) | 84.31 |
| N(4)-Pd(1)-N(1) | - | 98.0(7) | 96.87 |

**Table 4 (continued)**

| | [Pd(**HTE1PA**]⁺ 5 M HCl | [Pd(**HTE1PA**)]⁺ pH 3.5 | [Pd(**TE1PA**)]²⁺ pH 7 |
|---|---|---|---|
| Config. | *trans*-III | *trans*-I | *trans*-III |
| Pd(1)-N(1) | 2.059(5) | 2.080(5) | 2.009(10) |
| Pd(1)-N(2) | 2.044(5) | 2.035(5) | 1.998(12) |
| Pd(1)-N(3) | 2.057(5) | 2.038(5) | 2.007(10) |
| Pd(1)-N(4) | 2.046(6) | 2.045(5) | 2.050(12) |
| Pd(1)-N(5) | 5.015 | 3.066 | 5.002 |
| N(2)-H(2)---O(1) | - | 2.18(2) | - |
| N(1)-Pd(1)-N(2) | 85.49(18) | 84.98(19) | 84.2(5) |
| N(2)-Pd(1)-N(3) | 95.0(2) | 96.8(2) | 95.8(4) |
| N(3)-Pd(1)-N(4) | 85.2(2) | 84.8(2) | 84.5(5) |
| N(4)-Pd(1)-N(1) | 94.4(2) | 93.2(2) | 95.6(4) |

Crystals of [Pd(**cyclam**)](Cl)₂·6H₂O suitable for X-ray diffraction studies were obtained by slow evaporation from aqueous solution at pH 7. The structure evidences square-planar coordination with the Pd(II) ion located in the plane defined by the four nitrogen atoms. The hydrogen atoms of the two amino functions separated by a propylene bridge point to the same side of the N₄ plane, and opposite to the other two hydrogen atoms, resulting in a *trans-III* configuration according to the Bosnich's nomenclature. The two six-membered chelate rings adopt chair conformations (**Figure 1****)**. Crystals of [Pd(**TE1Bn**)](PdCl₄) were obtained by slow evaporation under heating of a solution of the complex in DMSO. The structure resembles that of the parent unsubstituted complex, [Pd(**cyclam**)]²⁺, in that a *trans*-III configuration is obtained with minimal distortion from a square-planar geometry (**Figure 2**). Crystals of [Pd(**TE1Py**)](PdCl₄) were obtained by slow evaporation of an aqueous solution of the complex at room temperature. While the crystal structure had a poor quality, it revealed a *trans-III* configuration similar to that obtained for the Pd-**TE1Bn** complex (**Figure 3**).

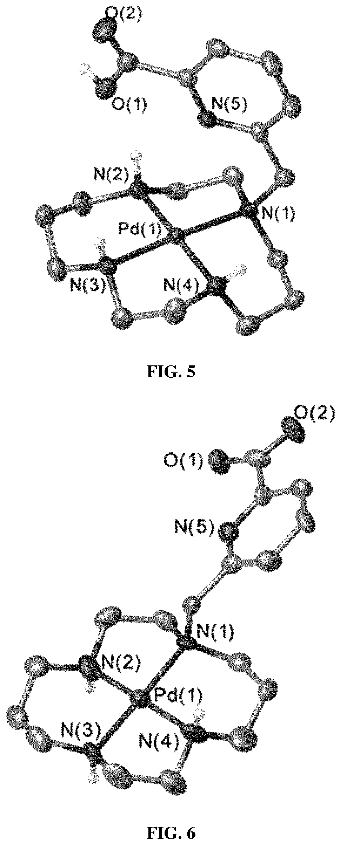

Three different crystal structures were obtained for the **TE1PA** complex by evaporation of aqueous solutions at different pH values, namely pH = 1, pH = 3.5 and pH = 7 (**Figures 4-6**). In each case, the Pd(II) ion is four-coordinated by the nitrogen atoms of the cyclam core. The nitrogen atom of the picolinate unit is not bound to palladium, regardless of whether the carboxylate function is protonated or not. The structure of [Pd(**HTE1PA)**](PdCl₄) obtained from a 5 M HCl aqueous solution shows a *trans*-III configuration (**Figure 4**), while interestingly [Pd(H**TE1PA**)](PdCl₄) obtained from an aqueous solution at pH 3.5 reveal a *trans-I* configuration (**Figure 5**). Crystals of [Pd(**TE1PA**)](ClO₄) 2H₂O obtained at pH 7 (by adjusting the pH with NaOH) after addition of NaClO₄ again indicate a structure with the *trans*-III configuration (**Figure 6**). The Pd-N bond lengths of the *trans*-III complex obtained in 5 M HCl are between 2.044(5)-2.059(5) Å, while those of the *trans*-III complex isolated at pH 7 are slightly shorter, ranging from 1.998(12) to 2.050(12) Å. The Pd(1)-N(1) bond, which involves the picolinate substituent, shows very different distances of 2.059(5) (5 M HCl) against 2.009(10) Å (complex at pH 7). A similar situation is observed for the Pd(1)-N(2) and Pd(1)-N(3) bonds, while the Pd(1)-N(4) distances are virtually identical. For the *trans-I* complex, the Pd(1)-N(1) distance of 2.080(5) Å is much longer than the Pd-NH distances of Pd(1)-N(2) at 2.035(5) Å, Pd(1)-N(3) at 2.038(5) Å, and Pd(1)-N(4) at 2.045(5) Å (**Table 4)**, but also much longer than the Pd(1)-N(1) distance in the *trans*-III complex. For both the *trans*-III and *trans-I* configurations, the angles characterizing the five-membered chelate rings (N(1)-Pd(1)-N(2) and N(3)-Pd(1)-N(4), < 86°) are lower than those involving six-membered chelate rings (N(2)-Pd(1)-N(3) and N(4)-Pd(1)-N(1), > 93°). In both *trans-III* complexes, the distance between the metal centre and the nitrogen atom of the pyridyl unit N(5) is > 5 Å, since the picolinate arm is orientated away from the central plane of the cyclam unit. On the contrary, in the *trans*-I configuration the protonated picolinate arm points towards the cyclam core, generating an intramolecular hydrogen bonding interaction between one NH group of the macrocycle and one oxygen atom of the carboxylic acid group (N(2)-H(2)•••••O(1), 2.18(2) Å).

### Example 2: Analytical assays

### Materials and methods

Reagents were purchased from Acros Organics^{®} or Sigma-Aldrich^{®} and used without further purification. Ultrapure water was freshly obtained from a Milli-Q dispenser. A solvent purification system was used to dispense dry solvents before each reaction. NMR spectra were recorded at the "Services communs" of the University of Brest. ¹H and ¹³C NMR spectra were recorded using Bruker Avance 500 (500 MHz), Bruker Avance 400 (400 MHz) or Bruker AMX-3 300 (300 MHz) spectrometers. Coupling constants are given in hertz and chemical shifts in ppm. Thermodynamic investigations were done at the "Instituto de Tecnologia Química e Biológica" (Oeiras, Portugal).

Thermodynamic studies: All UV-Vis and potentiometric titrations were performed at 25.0 °C in aqueous solutions containing 1.0 M of (K,H)Cl as background electrolyte. The potentiometric titration setup and methods have been previously described. The spectra of UV-Vis titrations were measured on a PerkinElmer lambda 650 spectrophotometer in Hellma QS-110 quartz cuvettes. The Pd(II) complexation by the studied ligands is complete in the range of pH useful for potentiometry, with complexation equilibria being only accessible at very low pH values where potentiometry cannot be used. For that reason, the complexation equilibria of the ligands with Pd(II) were studied at very low pH exclusively by UV-vis spectrophotometry, while potentiometry was used to study the protonation of the ligands as well as the protonation of the Pd(II) complexes; all experiments were run in the same ionic strength conditions. The overall equilibrium (formation) constants *β*_{MmHhL1} were determined in log units as defined by β_{MmHhLl} = [MₘHₕL₁]/[M]^{m}[H]^{h}[L]¹, while stepwise constants are defined by K_{MmHhLl} = [MmHₕL₁]/[MₘHₕ₋₁L₁][H] and are calculated from the difference in log units between overall constants of sequentially protonated species. The errors quoted are the standard deviations calculated by the fitting program for the experimental data in each system. The value of *K*_{w} = [H⁺][OH⁻] was taken from the literature as equal to 10^{-13.75} (Sweeton, F. H. et al., Journal of Solution Chemistry, 1974, Vol. 3, pp. 191-214).

For the UV spectrophotometry studies, the values of the stability constants of the Pd(II) chloride complexes (PdCl, PdCl₂, PdCl₃ and PdCl₄) were taken from the literature (Elding, L. I., Inorganica Chimica Acta, 1972, Vol. 6, pp. 647-651.). Considering these constants and the remaining experimental conditions used, we found that the species PdCl₄ is vastly dominant (*ca.* 93%) and thus its UV spectrum could be measured experimentally at pH ≈0 and inserted as a known spectrum in the fitting software. The complexation equilibria were studied in the range of HCl concentration of 0.02-1 M (pH = 0-1.5), additionally using a variable concentration of KCl to adjust the ionic strength in each point to 1.0 M. A set of 15 separate analytical points was prepared for the titration of each Pd(II)-ligand system, with a different concentration of HCl in each titration. All points contained fixed concentrations of Pd(II) (at 0.030 mM) and ligand (at 0.0315-0.0324 mM), and also a fixed total ionic strength at 1.0 M of (K,H)Cl, in a total volume of 5 mL per point. The spectrum of each ligand was also measured at the same concentration and conditions used in the titration points, with the pH adjusted to a value where all ligands are exclusively present as the H₂L species (pH ≈ 6.0) and thus may be inserted as a known spectrum for H₂L in the fitting software. The prepared titration points were incubated at 25°C to reach complete equilibrium, and their spectra were measured after 2 days in the case of Pd(II)-**TE1PA,** and 10 days in the case of Pd(II)-**TE1Py** and **Pd(II)-TE1Bn** which present slower complexation equilibria. The UV spectrophotometric data were fitted using the HypSpec 2008 software, from which it was possible to determine the equilibrium constant for the formation of the PdH₂L or PdHL species, respectively for **TE1PA** and **TE1Py.** These constants can then be used to determine the stability constants for the remaining complex species present in each equilibrium system. For **TE1Bn** it was not possible to determine the formation constant for the expected [Pd(**TE1Bn**)]²⁺ species, as it seems to be fully formed in the used experimental conditions. Nonetheless, analysis of the UV data suggests that this formation constant should be higher than *ca.* 43 since that is the lowest value for which it would be possible to determine a formation constant in the used conditions.

For the potentiometry experiments, the titrant was a KOH solution prepared at ca. 0.1 M from a commercial ampule of analytical grade, and its accurate concentration was obtained by application of the Gran's method upon titration of a standard HCl solution. A 0.100 M HCl titrant solution was also prepared by dilution of a standard commercial ampule of analytical grade. Ligand solutions were prepared at *ca.* 0.002 M, and a PdCl₂ solution was prepared at *ca.* 0.025 M from an analytical grade salt and standardized by complexometric titration. Sample solutions for titration contained *ca.* 0.04 mmol of ligand in a volume of 45 mL, and in Pd(II) complexation titrations the PdCl₂ solution was added at 0.8 equivalents of the ligand amount. The Pd(II) titrations were first run in the forward direction (acidic to basic) with KOH, and then the same sample was titrated backward with HCl to check if equilibrium was attained. The electromotive force of the sample solutions was measured after calibration of the electrode by titration of a standard solution containing 0.05 mmol of HCl in 1.0 M KCl medium. Each potentiometric titration consisted of 90-140 equilibrium points in the range of pH 2.5-11.5 for protonations or pH 2.5-10 for complexations. The potentiometric data were fitted using the Hyperquad 2008 software. The titrations containing Pd(II) for **TE1PA** and **TE1Py** display only the protonation equilibria of the Pd(II) complexes, since these are fully formed at pH ≥ 2.0. To calculate the protonation constants of both complexes, the backward titrations were adopted since they seemed slightly more stabilized than the forward ones especially for TE1Py. To fit these potentiometric data, the equilibrium constant obtained by UV-vis spectrophotometry for the formation of the [Pd(H₂**TE1PA**)]³⁺ or [Pd(**HTE1Py**)]³⁺ species was inserted as a fixed value in the equilibrium model of the corresponding titration, and only the values of the equilibrium constants for the remaining complex species were refined. The species distribution diagrams were plotted considering 1 mM total concentration of both the ligand and Pd(II), using the HySS software.

Acid-mediated dissociation: The acid-assisted dissociation of [Pd(**cyclam**]²⁺ and [Pd(**TE1PA**)]⁺ complexes was investigated by ¹H NMR in 5 M DCl at 90°C (each NMR sample was incubated at 90°C all along the experiment: 5 months). The concentration of sample solutions containing each complex was of 0.05M per sample. The dissociation was observed by recording a ¹H NMR spectrum every day during the first weeks, then each week. The signals of the protons of the macrocycles were followed and dissociation deducted thanks to the difference of ¹H NMR spectra between the ligands **(cyclam** and **TE1PA)** and their corresponding Pd(II) complexes.

### Results

### Thermodynamic studies (chelate formation)

In order to enable a detailed study of the complexation properties of Pd(II) with monosubstituted cyclam derivatives, a combination of UV spectrophotometry and potentiometry performed in aqueous medium at 25 °C was used. To overcome the expected high values of the formation constants for the complexes, the UV titrations were run in a very acidic solution (pH = 0-1.5) at a 1.0 M concentration of (K,H)Cl electrolyte. In such low pH conditions, Pd(II) complexation could be observed for both **TE1PA** and **TE1Py**. Fitting of the UV spectroscopy data then allowed for determination of the formation constant for the protonated species [Pd(H₂**TE1PA**)]³⁺ or [Pd(**HTE1Py**)]³⁺ with subsequent establishment of the remaining equilibrium constants for the existing species (constants were fitted to the potentiometric titration of the corresponding system). No formation constant for any complex species of **TElBn** could be determined as the complex appears to be fully formed under the used experimental conditions. However, from the UV data it can be inferred that its log K value should be higher than *ca.* 43. The ligand protonation equilibria were studied by potentiometry in 1.0 M KCl aqueous medium, as were the protonation equilibria of the Pd(II) complexes since these are fully formed at pH ≥ 2.0. The protonation and complexation constants are presented in **Table 5** and **Table 6** below.

**Table 5**

| **Equilibrium reaction ^{a}** | **TE1PA** | **TE1Py I** | **TElBn** |
|---|---|---|---|
| L + H⁺ ⇄HL | 11.13(1) | 11.50(1) | 11.31(1) |
| HL + H⁺ ⇄H₂L | 10.06(1) | 10.39(1) | 9.39(1) |
| H₂L + H⁺ ⇄H₃L | 3.05(2) | 2.65(1) | 2.21(1) |
| H₃L + H⁺ ⇄H₄L | 2.28(3) | 2.83(1) | 2.89(2) |
| H₄L + **H⁺** ⇄H₅L | 1.69(8) | - | - |
| Pd²⁺ + L ⇄PdL | 38.4(1) | 42.6(1) | > 43 |
| PdL + H⁺ ⇄PdHL | 2.9(1) | 1.6(1) | - |
| PdHL + H⁺ ⇄PdH₂L | 1.8(1) | - | - |

**Table 6**

| Equilibrium reaction ^{a} | **TE1PA** | **TE1Py** | **TE1Bn** |
|---|---|---|---|
| L + H⁺ ⇄HL | 11.13(1) | 11.50(1) | 11.31(1) |
| H + 2 H⁺ ⇄ H₂L | 21.19(1) | 21.89(1) | 20.70(1) |
| H + 3 H⁺ ⇄ H₃L | 24.24(2) | 24.55(1) | 22.91(2) |
| H + 4 H⁺ ⇄ H₄L | 26.51(2) | 27.38(1) | 25.80(1) |
| H + 5 H⁺ ⇄H₅L | 28.21(6) | - | - |
| Pd²⁺ + L ⇄PdL | 38.4(1) | 42.6(1) | >43 |
| Pd²⁺ + L + H⁺ ⇄PdHL | 41.3(1) | 44.2(1) ^{b} | - |
| Pd²⁺ + L + 2 H⁺ ⇄PdH₂L | 43.1(1) ^{b} | - | - |

| | | | |
|---|---|---|---|
| ^{a} charges are omitted for clarity, ^{b} determined by UV spectrophotometry. | | | |

The protonation constants of the ligands display the usual pattern found for cyclam derivatives of two basic protonation centers while the remaining ones are more acidic. The most notorious feature, consistent with **cyclam,** is that both **TE1Py** and **TE1Bn** show the uncommon inversion of values for two acidic protonation centers where the third constant is lower than the fourth. This feature may be attributed to the increased strain exhibited upon addition of the third proton to the cyclam skeleton. For the Pd(II) complexes, using the previously UV-determined formation constants to fit the potentiometric data, it was found that **TE1PA** shows a very high stability constant (log *K*_{ML} = 38.4), while **TE1Py** displays an even higher constant (log *K*_{ML} = 42.6). Both values are much higher than for the literature tetra(2-hydroxyethyl) cyclam derivative (log *K*_{ML} = 18.32) **(**Hay, R. W. et al., Journal of the Chemical Society, Dalton Transactions, 1987, pp. 2605-2613), despite still being significantly lower than the value estimated for cyclam itself (log *K*_{ML} = 56.9) (Harrington, J. M. et al., Inorganica Chimica Acta, September 2005, Vol. 358, pp. 4473-4480).

### Acid-mediated dissociation ("cold" inertness)

The slow dissociation of the complexes is one of the most important features to be evaluated for the selection of medically relevant compounds. The acid-assisted dissociations of the cyclam and TE1PA complexes were studied in acidic aqueous solutions. The dissociation was monitored using ¹H NMR by following the changes of the complexes at 90 °C in 5 M DCl.

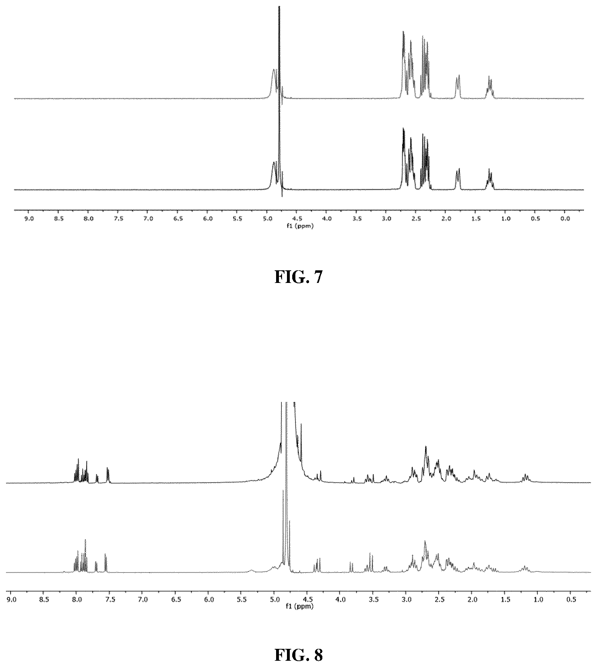

The unchanged spectra for **cyclam** and **TE1PA** complexes **(****Figure 7** and **Figure 8** respectively) unambiguously confirmed the absence of evolution of the structures even after five months incubation with constant stirring. This evidences the perfect matching of both **cyclam** and **TE1PA** ligands with Pd(II), and further that the presence of the "picolinate" pendant in **TE1PA** does not compromise kinetic inertness. As shown previously **(Example 1,** X-ray structures), the "picolinate" pendant in **TE1PA** is not involved in the metal coordination scheme.

### Example 3: Radiolabeling assay

### Material and methods

Reagents were purchased from Acros Organics^{®} or Sigma-Aldrich^{®} and used without further purification. Ultrapure water was freshly obtained from a Milli-Q dispenser. A solvent purification system was used to dispense dry solvents before each reaction. NMR spectra were recorded at the "Services communs" of the University of Brest. ¹H and ¹³C NMR spectra were recorded using Bruker Avance 500 (500 MHz), Bruker Avance 400 (400 MHz) or Bruker AMX-3 300 (300 MHz) spectrometers. Coupling constants are given in hertz and chemical shifts in ppm. The radiolabeling experiments were performed at the South African Nuclear Energy Corporation in Pretoria.

¹⁰⁹Pd-radiolabeling: The production and purification of ¹⁰⁹Pd-isotope was completed according to the method previously described (Das, T. et. al., Radiochimica Acta, July 2008, Vol. 96, pp. 427-433). An isotopically enriched palladium metal target (94.6% in ¹⁰⁸Pd, 1.0 - 1.3 mg) (Isoflex USA, San Francisco, USA) underwent thermal neutron irradiation (flux of 1×10¹⁴ n/cm².s¹) for 30-36 h in the high-flux neutron reactor, SAFARI-1, at Necsa, South Africa. The irradiated target was dissolved by heating in aqua regia (1 mL) and then evaporated to dryness. Excess HNO₃ was removed by further addition and evaporation of c. HCl (1 mL). The residue was redissolved in 6 M HCl (1 mL) to form [¹⁰⁹Pd][PdCl₄]²⁻ as a dark yellow/orange solution. The radionuclide impurity formed during irradiation, silver-111, was precipitated out of solution by addition of AgNO₃ solution in 0.1 M HNO₃ ([20 mg/mL], 100 µL). The mixture was heated for 5 min at 50 - 60°C, left to cool, vortexed and then centrifuged (6000 rpm, 5 min) using a Hettich EBA 20 centrifuge (A. Hettich GmbH & Co, Tuttlingen, Germany). The supernatant was carefully transferred to a clean vial and dried under a stream of argon. The residue was redissolved in 0.8 M HCl (0.52 mL) for use in radiolabeling reactions. The radionuclidic purity of the ¹⁰⁹Pd-solution was determined by high resolution gamma ray spectrometry using a calibrated HPGe Detector (Canberra GC2518 (25% efficiency) (Mirion Technologies, Atlanta, USA). For radiolabeling, all solvents used were HPLC grade (Merck KGaA, Darmstadt, Germany,) and all buffer solutions were prepared using Milli-Q grade water (>18 MΩ/cm). The activity of the [¹⁰⁹Pd][PdCl₄]²⁻ solution and ¹⁰⁹Pd-chelator complexes were measured using a Capintec CRC-15R gamma detector (Capintec Inc., Florham Park, NJ, USA) (isotope measurement number 435 × 10). All radiolabeled compounds were analyzed by radio-HPLC analysis on Varian Prostar 325 UV-Vis HPLC apparatus (Varian Inc., Walnut Creek, CA, USA) fitted with a radiometric GABI Star gamma detector (raytest GmbH, Straubenhardt, Germany). The radio-HPLC was run using a Zorbax Stable Bond-C18 column (5 µm; 4.6 × 250 mm) with a gradient elution over 20 min of 95-5% A in B (Solvent A = H₂O (0.1% TFA); Solvent B = MeCN (0.1% TFA)). [¹⁰⁹Pd][PdCl₄]²⁻ solution (10 µL; 10 - 40 MBq;) was added to the appropriate volume of chelator (1 mg/mL, molar equivalent ligand:metal = 2:1) dissolved in NH₄OAc buffer (0.1 M) with extra NH₄OAc (0.1 M) added to ensure a final reaction volume of 300 µL. The pH of the solution was adjusted to either pH 3.5, 7 or 8.5 and the radiolabeling reaction was then kept at 25°C or 90°C for 10 or 30 minutes. Radio-HPLC analysis was performed on the reaction solution in order to determine the radiolabeling efficiency (%).

EDTA challenge: **TE1PA** and **cyclam** were radiolabeled as described hereinabove except for the use of Phosphate-Buffered Saline (PBS) buffer (0.01 M, pH 7) rather than 0.1 M NH₄OAc. The [¹⁰⁹Pd]PdCl₄²⁻ solution (10 µL, 16 MBq for cyclam and 17 MBq for TE1PA) was added to the chelator solution (chelator: metal 2:1) and the pH adjusted to 7. The reaction was done at 90°C for 30 minutes to allow the maximum complexation. Radio-HPLC analysis was done to confirm labelling **([¹⁰⁹Pd]Pd-TE1PA** = 98% LE; **[¹⁰⁹Pd]Pd-cyclam** = 99% LE) followed by addition of EDTA solution (2.5M, 480 µl, 1000 eq) to each reaction vial. The reaction solutions were left at room temperature (25°C) with radio-HPLC monitoring at 1 h, 2 h and 24 h.

### Results

### ¹⁰⁹Pd-radiolabeling (radiolabeling efficiency and "hot" inertness)

All cyclam derivatives were studied for complexation of the ¹⁰⁹Pd-radioisotope. The aim was to compare the macrocycles, and specifically **TE1PA** and **cyclam,** in terms of their ¹⁰⁹Pd-labelling efficiency (the kinetic process of complexation) and inertness under radiolabeling conditions for potential application in developing new palladiumbased radiopharmaceuticals.

The ¹⁰⁹Pd-radionuclide was produced with a specific activity of 2.9 GBq/mg (78 mCi/mg) from a 94% enriched ¹⁰⁸Pd-target following high-flux neutron beam irradiation. After the precipitation and separation of Ag-radionuclide impurities from the processed [¹⁰⁹Pd][PdCl₄²⁻] solution, gamma ray spectroscopy (88 keV (gamma from ^{109m}Ag-daughter nuclide), 311, 413, 602, 636, 647, 781 keV) indicated a radionuclidic purity > 99.9% with a radiochemical yield of 90%. In order to compare the efficiency of the different chelators for ¹⁰⁹Pd-complexation, the radiolabeling reactions were performed in 0.1 M NH₄OAc at different pH values (pH 3.5, 7 and 8.5), two temperatures (25 and 90 °C), and different reaction times (10 and 30 min). The reactions were then analyzed by radio-HPLC to determine the radiolabeling efficiency (% LE).

The radiometal complexation for all chelators was very good (> 90% LE) at pH 3.5 and 90 °C over 30 min. The relatively long reaction time at this temperature provided enough energy to activate metal-chelator bond formation and to overcome the kinetic process of complexation to all four ligands. For comparison purposes, the radiolabeling efficiencies were evaluated at different temperatures over the shorter reaction time of 10 min.

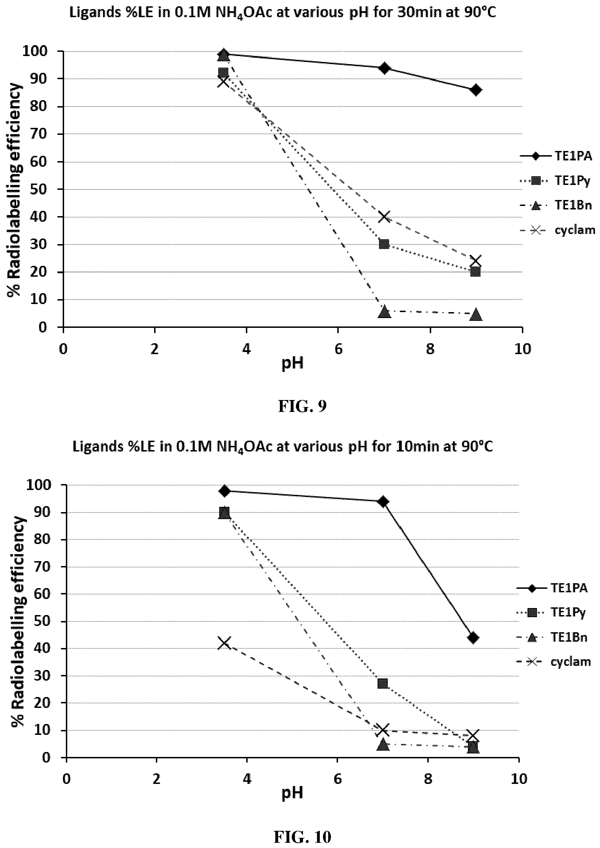

The results are presented in **Figures 9-12** and **Table 7** below.

**Table 7: ¹⁰⁹Pd-Radiolabelling efficiency LE (%) determined at pH 3.5 in 0.1 M NH₄OAc aqueous solution.**

| | **cyclam** | **TE1Bn** | **TE1Py** | **TE1PA** |
|---|---|---|---|---|
| | %LE | %LE | % LE | %LE |
| 30 min, 90°C | 89 | 99 | 92 | 98 |
| 10 min, 90°C | 42 | 90 | 90 | 98 |
| 10 min, 25°C | 38 | 70 | 78 | 96 |

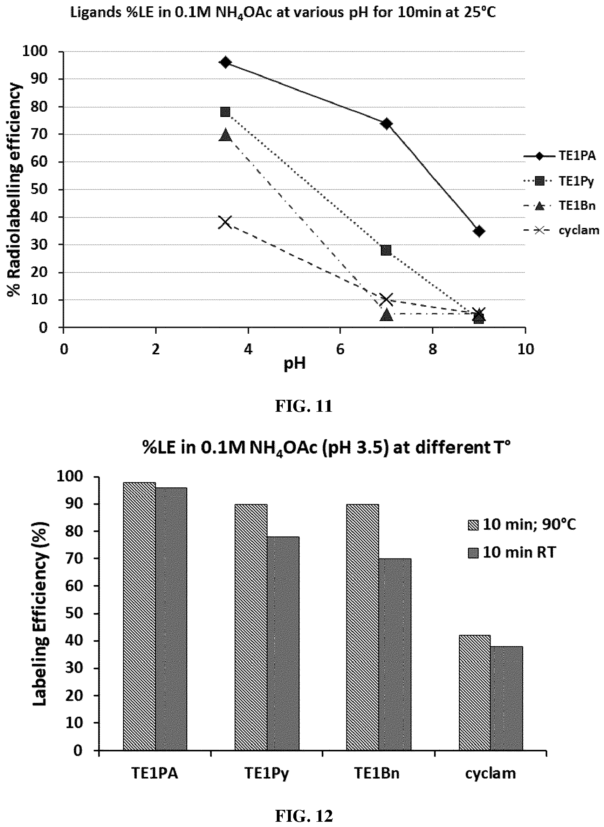

With 99% LE at 90 °C and 96% at room temperature within 10 min, **TE1PA** clearly presented the best efficiency for complexation of ¹⁰⁹Pd-nuclide among the four chelators. While heating generally improved the radiolabeling efficiency, the results presented on **Figure 12** clearly shows that the functionalization of the cyclam backbone accelerates the radiolabeling, especially looking at the results at room temperature.

Indeed, the poor LE obtained for **cyclam** is evidence of slow complexation kinetics, particularly at short radiolabeling times, and longer times (> 30 min) and high temperature are required to improve complexation (≥ 90% LE). The differences between LEs of **TE1Bn, TE1Py**, and **TE1PA** at 90 °C are not very significant. However, these chelators show rather different performances at room temperature (96% for **TE1PA** vs 78% and 70% for **TE1Py** and **TE1Bn,** respectively). Without being bound by any theory, the Applicant believes that the picolinate moiety at pH 3.5 may be rather efficient in chelating Pd(II) close to the macrocyclic cavity, perhaps assisted by the intramolecular hydrogen bond observed in the X-ray structure (N(2)-H(2)·····O(1)). This intermediate could then evolve to the final complex upon coordination of Pd(II) to the N atoms of the macrocycle.

The results for labelling experiments performed at other pH values (7.0 and 8.5) evidence a noticeable drop in radiolabeling efficiency for all chelators with respect to pH 3.5, except for **TE1PA.** Under these radiolabeling conditions, the [¹⁰⁹Pd][Pd(**TE1PA)**]⁺ complex is formed with a very high radiolabeling yield as compared to the other chelators, (LE > 85% for **TE1PA,** and < 40% for **cyclam, TElBn** and **TE1Py**; 90 °C, 30 min). Even at a pH of 8.5 at room temperature with a short reaction time, the LE of **TE1PA** stay moderate (35%) as compared to the LE of the other chelators that falls to values < 5%.

### EDTA challenge

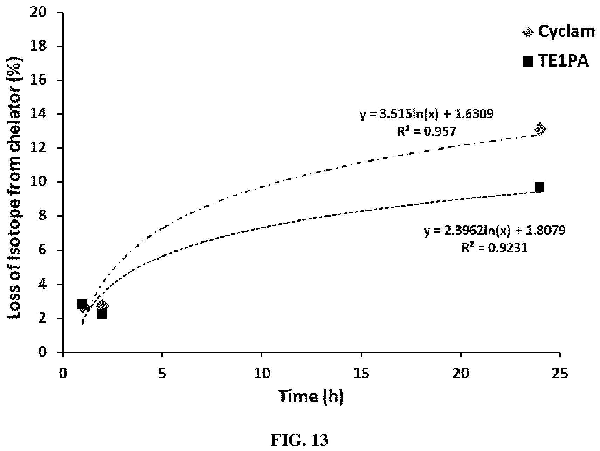

To further investigate and compare the radiolabeled complex inertness with regard to transchelation, an EDTA challenge was performed under physiological pH conditions in PBS buffer (0.01 M, pH 7). **TE1PA** and **cyclam** were radiolabeled with palladium-109 in PBS buffer at 90 °C to obtain maximum radionuclide complexation (> 98%) and then challenged by an EDTA solution (1000 eq) at room temperature. Transchelation to EDTA was then monitored by radio-HPLC over 24 h (**Figure 13**).

Not much transchelation was observed at the early time points with the behavior of both radio-complexes being quite similar (only 2-3% loss of the radiometal). However, a significant improvement in inertness of [¹⁰⁹Pd][Pd(**TE1PA)**]⁺ over [¹⁰⁹Pd][Pd(**cyclam)**]²⁺ is observed after 24h at room temperature, ultimately demonstrating the enhanced properties of the picolinate ligand (**TE1PA**).

### Example 4: Comparison of Cu(II) and Pd(II) chelates

### Experimental data

Information from the literature as well of the data disclosed in the present application ("unpublished") regarding **cyclam** and **TE1PA** chelates of Cu(II) and Pd(II) are presented in **Table 8** below for comparison purposes.

**Table 8**

| **Chelate** | **Analytical data ("cold" cation)** | **Radiolabeling ("hot" cation)** |
|---|---|---|
| [Cu(**cyclam)**]²⁺ | **Thermodynamic** | |
| | 25 °C with I = 0.1 M in KCl | **⁶⁴Cu radiolabeling** |
| (*cf.* list of references below^{∗}) | log K_{ML} = 28.09 | pH: 5.5 |
| | pM = 21.41 | 20-25°C, 10 min: 97% |
| | **Dissociation** | **Inertness** |
| | 5M HCl, 25°C: 21 min | {No data found in literature} |
| | 5M HCl, 90°C: < 3 min | |
| | 10M HCl, 90°C: < 3 min | |
| [Pd(**cyclam**)]²⁺ | **Thermodynamic** | **¹⁰⁹Pd radiolabeling** |
| | 25°C in 0.1 M NaClO₄ | pH 3.5 in 0.1 M NH₄OAc |
| | log K_{ML} = 56,9 | 90°C, 30 min: 89% |
| | (HARRINGTON, J. M. et al., Inorganica Chimica Acta, September 2005, Vol. 358, pp. 4473-4480.) | 90°C, 10 min: 42% |
| | | 25°C, 10 min: 38% |
| | | (Unpublished) |
| | **Dissociation** | **Inertness** |
| | HCl 5M, 90°C: Inert | 1000 EDTA, 25°C, 24h:eq. |
| | | 13% demetallation |
| | (Unpublished) | (Unpublished) |
| [Cu(**TE1PA**)]⁺ | **Thermodynamic** | **⁶⁴Cu radiolabeling** |
| | 0.10 M using KNO₃ | 0.1 M NH₄OAc |
| | log K_{ML} = 25.5 | 25°C, 15 min: 99% |
| | pM= 18.84 | (FRINDEL, L et al., Nuclear Medicine and Biology, Volume 41, Supplement, May 2014, pp. e49-e57) |
| | (LIMA, L. M. P. et al., Inorganic Chemistry, May 2012, Vol. 51, No. 12, pp. 6916-6927) | |
| | **Dissociation** | |
| | HCl 1M, 25°C: 32 min (LIMA *et al*., 2012) | |
| [Pd(**TE1PA**)]²⁺ | **Thermodynamic** | **¹⁰⁹Pd radiolabeling** |
| | 25°C in 1.0 M KCl | pH 3.5 in 0.1 M NH₄OAc |
| | log K_{ML} = 38.4 | 90°C, 30 min: 98% |
| | (Unpublished) | 90°C, 10 min: 98% |
| | | 25°C, 10 min: 96% |
| | **Dissociation** | (Unpublished) |
| | HCl, 5M, 90°C: Inert | |
| | (Unpublished) | **Inertness** |
| | | 1000 eq. EDTA, 25°C, 24h: |
| | | 9% demetallation |
| | | (Unpublished) |

| | | |
|---|---|---|
| ^{∗}List of references for [Cu(**cyclam**)]²⁺: (HEROUX, K. J. et al., Dalton Transactions, April 2007, No. 21, pp. 2150-2162; MOTEKAITIS, R. J et al., Journal of Inorganic Chemistry, June 1996, Vol. 35, pp. 3821-3827; KODAMA, M. and Kimura, E., Journal of the Chemical Society, Dalton Transactions, 1976, No. 2, pp. 116-120; LAN, W.-J. and Chung, C.-S., Journal of the Chemical Society, Dalton Transactions, 1994, No. 2, pp. 191-194; SMITH, S. V., Journal of Inorganic Biochemistry, Vol. 98, No. 11, September 2004, pp. 1874-1901; BLOWER, P. J. et al., Nuclear Medicine and Biology, Vol. 23, No. 8, July 1996, pp. 957-980). | | |

### Discussion

The experimental data presented on the above **Table 8** clearly shows that the inertness of the **cyclam** and **TE1PA** chelates of the nonradioactive Pd(II) ("cold" cation) is far superior to the inertness of the corresponding Cu(II) chelates. This is deemed to be unexpected, as no information whatsoever was available in the literature regarding the inertness of the Pd(II) chelates. The Applicants surprisingly found out that neither [Pd(**cyclam**)]²⁺ nor [Pd(**TE1PA**)]²⁺ present any significant dissociation in the tested conditions (HCl 5M, 90°C), in sharp contrast with the stability of [Cu(**cyclam**)]²⁺ and [Cu(**TE1PA**)]²⁺ described in the art. However, although quite limited, the inertness of the Cu(II) chelates remains within appropriate working range for applications in diagnosis.

This inertness of [Pd(**cyclam**)]²⁺ and [Pd(**TE1PA**)]²⁺ was also confirmed in "hot" conditions, wherein the cation is radioactive ¹⁰⁹Pd. For Pd(II) radiolabeling, the **TE1PA** chelate is slightly more inert than the **cyclam** chelate (9% and 13% demetallation respectively, *i.e*., the former is more inert than the latter), although this difference may not be very significant in practice. Again, no information whatsoever was available in the literature regarding the stability of radioactive Pd(II) chelates with a cyclam-based ligand.

Moreover, it was surprisingly evidenced by the Applicants that the suitability of **cyclam** for radiolabeling dramatically falls when replacing radioactive Cu (⁶⁴Cu) by radioactive Pd (¹⁰⁹Pd), especially at low temperature. Indeed, the high radiolabeling rate of 97% for [⁶⁴Cu(**cyclam**)] drops to 38% for [¹⁰⁹Pd(**cyclam**)] (25°C, 10 min) and, even at higher temperatures, [¹⁰⁹Pd(**cyclam**)] is slowly and sparingly obtained (90°C, 10 min: 42%). By contrast, when **TE1PA** is used as ligand instead of **cyclam**, no significant loss of radiolabeling performance is observed between [⁶⁴Cu(**TE1PA**)] and [¹⁰⁹Pd(**TE1PA**)]: on the contrary a very high yield (LE) is quickly obtained even in mild conditions (25°C, 10 min: 96%). Therefore, **TE1PA** is especially advantageous over **cyclam** as a ligand for theranostic applications.

Consequently, the outstanding properties of Pd(II) chelates [Pd(**TE1PA**)] and [¹⁰⁹Pd(**TE1PA**)] are fully unexpected in view of the prior art disclosing Cu(II) and Pd(II) **cyclam** chelates. These properties make them very attractive for therapeutic use, in particular in radiotherapy.

## Claims

1. Chelate resulting from the complexation of a palladium(II) cation with a ligand of formula (I) 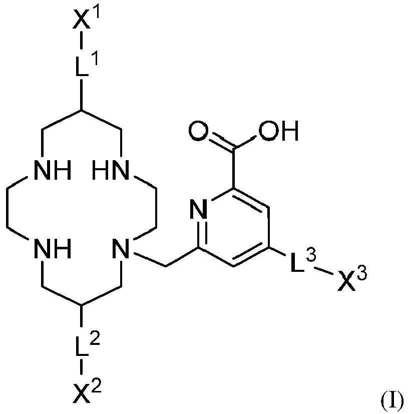
or a pharmaceutically acceptable salt and/or solvate thereof; wherein
**L¹, L²** and **L³** each independently represents:
- a single bond; or
- a linker selected from alkyl, arylalkyl, alkylaryl-alkyl, heteroalkylaryl-alkyl, alkenylaryl-alkyl, alkynylaryl-alkyl, heteroarylalkyl, alkylheteroaryl-alkyl, heteroalkylheteroaryl-alkyl, alkenylheteroaryl-alkyl, alkynylheteroaryl-alkyl, heteroalkyl, arylheteroalkyl, alkylaryl-heteroalkyl, heteroalkylaryl-heteroalkyl, alkenylaryl-heteroalkyl, alkynylaryl-heteroalkyl, heteroarylheteroalkyl, alkylheteroaryl-heteroalkyl, heteroalkylheteroaryl-heteroalkyl, alkenylheteroaryl-heteroalkyl, alkynylheteroaryl-heteroalkyl, aryl, alkylaryl, heteroalkylaryl, alkenylaryl, alkynylaryl, heteroaryl, alkylheteroaryl, heteroalkylheteroaryl, alkenylheteroaryl, alkynylheteroaryl, alkenyl, arylalkenyl, alkylaryl-alkenyl, heteroalkylaryl-alkenyl, alkenylaryl-alkenyl, alkynylaryl-alkenyl, heteroarylalkenyl, alkylheteroaryl-alkenyl, heteroalkylheteroaryl-alkenyl, alkenylheteroaryl-alkenyl, alkynylheteroaryl-alkenyl, alkynyl, arylalkynyl, alkylaryl-alkynyl, heteroalkylaryl-alkynyl, alkenylaryl-alkynyl, alkynylaryl-alkynyl, heteroarylalkynyl, alkylheteroaryl-alkynyl, heteroalkylheteroaryl-alkynyl, alkenylheteroaryl-alkynyl, alkynylheteroaryl-alkynyl,
wherein said linker is optionally interrupted or terminated by at least one coupling product selected from triazolyl, NH(C=O), S-S, OCH₂CH(OH), NHCH₂CH(OH), SCH₂CH(OH), N=CH, NH(C=O)NH, NH(C=S)NH, (C=O)CH₂S, S(C=O)O, CH₂CH₂S, O(C=O)NH, SCH₂(C=O), NH(C=O), C(=O)O, (C=O)O(C=O), O, S, NHC(NH²⁺)CH₂, NHNH=CH, C=C and C≡C;
**X¹**, **X²** and **X³** each independently represents:
- a hydrogen atom;
- a coupling function selected from amine, isothiocyanate, isocyanate, activated ester, carboxylic acid, activated carboxylic acid, alcohol, alkene, trans-cyclooctene (TCO), alkyne, bicyclo[6.1.0]nonyne (BCN), dibenzocyclooctyne (DBCO), halide, azide, siloxy, phosphonic acid, thiol, tetrazine, norbornen, oxoamine, aminooxy, thioether, haloacetamide glutamate, glutaric anhydride, succinic anhydride, maleic anhydride, aldehyde, ketone, hydrazide, chloroformate, and maleimide; or
- a bioactive group selected from antibody, peptide, protein, lipid, monosaccharide, polysaccharide, fatty acids, hapten, liposomes, polyamine, fluorophore, chromophore, macrocyclic ligand, nanoparticle, polymeric microparticle, cationic group suitable for cellular internalization, and a combination thereof.

2. The chelate according to claim **1**, wherein said palladium(II) cation is a radioisotope of palladium(II).

3. The chelate according to claim **2**, wherein said radioisotope of palladium(II) is selected from ¹⁰³Pd, ¹⁰⁹Pd, ¹⁰⁰Pd, ¹⁰¹Pd, ¹⁰⁷Pd, ^{111m}Pd, and ¹¹²Pd; preferably selected from ¹⁰³Pd and ¹⁰⁹Pd.

4. The chelate according to any one of claims **1** to **3**, wherein **L¹** and/or **L²** represents a linker selected from alkyl, arylalkyl and alkylaryl-alkyl; preferably a linker selected from -(CH₂)ₘ-, -phenyl-(CH₂)ₙ- or -(CH₂)ₚ-phenyl-(CH₂)ₙ-; wherein **m** is an integer ranging from 1 to 12, **n** is an integer ranging from 1 to 6 and **p** is an integer ranging from 1 to 6;
wherein said linker is optionally interrupted or terminated by at least one coupling product as defined in claim **1**.

5. The chelate according to any one of claims **1** to **4**, wherein **X¹**, **X²** and/or **X³** represents a bioactive group as defined in claim **1**; preferably a bio-vectorizing group selected from antibody, peptide, protein, lipid, monosaccharide, polysaccharide, fatty acids, hapten, liposomes, polyamine, macrocyclic ligand, nanoparticle, polymeric microparticle, cationic group suitable for cellular internalization, and a combination thereof.

6. The chelate according to any one of claims **1** to **5**, wherein **X¹**, **X²** and/or **X³** represents a coupling function as defined in claim **1**.

7. The chelate according to any one of claims **1** to **6**, wherein at least one group selected from **X¹**, **X²** and **X³** represents a bioactive group as defined in claim **1** and at least one group selected from **X¹**, **X²** and **X³** represents a coupling function as defined in claim **1.**

8. The chelate according to any one of claims **1** to **7**, wherein -**L¹**-**X¹**, -**L²**-**X²** and/or -**L³**-**X³** represents a single bond and a hydrogen atom, preferably -**L¹-X¹** and/or -**L²**-**X²** represents a single bond and a hydrogen atom, more preferably -**L²**-**X²** represents a single bound and a hydrogen atom.

9. The chelate according to any one of claims **1** to **8**, wherein said ligand is 6-((1,4,8,11-tetraazacyclotetradecan-1-yl)methyl)picolinic acid (**HTE1PA**) or a pharmaceutically acceptable salt and/or solvate thereof.

10. Pharmaceutical composition comprising a chelate according to any one of claims **1** to **9** and at least one pharmaceutically acceptable carrier.

11. The chelate according to any one of claims **1** to **9** or the pharmaceutical composition according to claim **10** for use as a medicament.

12. The chelate according to any one of claims **1** to **9** or the pharmaceutical composition according to claim **10** for use in a method of diagnosis *"in vivo"* and/or in the treatment of a proliferative disease.

13. Use of a chelate according to any one of claims **1** to **9** or a pharmaceutical composition according to claim **10** in biological imaging, preferably in medical imaging.

14. Use of a ligand as described in any one of claims **1** to **9** in the manufacture of a chelate according to any one of claims **1** to **9.**

15. Process for manufacturing a chelate according to any one of claims **1** to **9**, wherein said process comprises one of the following steps:
(a1) a step of contacting said ligand with a stoichiometric amount of sodium tetrachloropalladate in refluxing water at a pH ranging from about 3 to about 5;
(a2) a step of contacting a solution of said ligand with a solution of palladium(II) chloride in an aqueous medium at a temperature ranging from about 20°C to about 30°C, and at a pH ranging from about 0 to about 2.5; or
(a3) a step of contacting a solution of said ligand with a solution of [¹⁰⁹PdHPdCl₄]²⁻ at a temperature ranging from about 20°C to about 30°C, and at a pH ranging from about 2.5 to about 7.5.
